# EUROPÄISCHE PATENTSCHRIFT

(11) **EP 0 823 898 B1**
(45) Veröffentlichungstag und Bekanntmachung des Hinweises auf die Patenterteilung: **26.02.2003**
(21) Anmeldenummer: 96914914.5
(22) Anmeldetag: 15.04.1996
(51) Int. Cl.: C07D 233/76, C07D 233/80

(54) **HYDANTOINDERIVATE ALS ZWISCHENPRODUKTE FÜR PHARMAZEUTISCHE WIRKSTOFFE**
HYDANTOINE DERIVATIVES AS INTERMEDIATE PRODUCTS FOR ACTIVE PHARMACEUTICAL AGENTS
DERIVES HYDANTOINE UTILISES COMME PRODUITS INTERMEDIAIRES POUR PRINCIPES ACTIFS PHARMACEUTIQUES

(30) Priorität: 28.04.1995 DE 19515177
(43) Veröffentlichungstag der Anmeldung: 18.02.1998
(73) Patentinhaber: HOECHST AKTIENGESELLSCHAFT, 65929 Frankfurt am Main (DE)
(72) Erfinder: ZOLLER, Gerhard, D-61137 Schöneck (DE); KLINGLER, Otmar, D-63110 Rodgau (DE); KNOLLE, Jochen, D-65830 Kriftel (DE); STILZ, Hans, Ulrich, D-65929 Frankfurt am Main (DE); WEHNER, Volkmar, D-97657 Sandberg (DE)
(86) Internationale Anmeldenummer: EP9601572
(87) Internationale Veröffentlichungsnummer: WO96033976

(56) Entgegenhaltungen:
- WO-A-95/14008
- DE-A- 4 338 944
- DE-A- 4 427 979
- US-A- 5 389 614
- J. CHEM. SOC., 1960, Seiten 2994-3001, XP002013107 CONNORS, T., A. ET AL.: "Aryl-2-halogenalkylamines. Part XIX. Some NN-Di-chloroethylaminophenyl- and -phenylalkylhydantoins and Related Amino-acids."

## Beschreibung

Die vorliegende Erfindung betrifft Hydantoinderivate der allgemeinen Formel I, 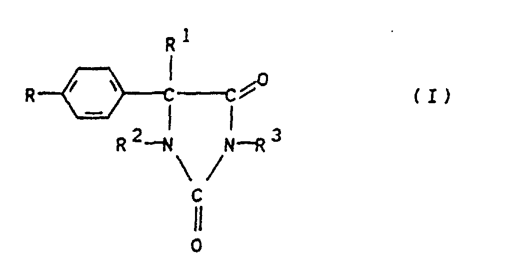 die wertvolle Zwischenprodukte für die Herstellung von pharmazeutischen Wirkstoffen darstellen, ihre Herstellung und ihre Verwendung bei der Herstellung der Wirkstoffe.

In J. Chem. Soc. 1960, 2994 - 3007, ist das racemische 5-(p-Acetylaminophenyl)-5-methylhydantoin beschrieben, also die Verbindung der Formel I, in der R für NH-CO-CH₃, R¹ für Methyl und R² und R³ für Wasserstoff stehen. In der US-A-5389614, der DE-A-4338944 und der DE-A-4427979 (deutsche Patentanmeldungen P4338944 und P4427979) und der WO-A-95/14008 (PCT-Anmeldung PCT/EP94/03491) sind substituierte 5-Ring-Heterocycien beschrieben, die die Zell-Zell-Adhäsion, insbesondere z.B. die Thrombocytenaggregation, hemmen. Diese Wirkstoffe enthalten einen mehrfach substituierten Imidazolidin-, Oxazolidin-, Thiazolidin- oder Pyrrolidinring, der über eine zweiwertige Gruppe, z.B. über eine Alkylengruppe oder über verschiedene andere Gruppen, mit einer unsubstituierten oder substituierten Amino-, Amidino- oder Guanidinogruppierung verknüpft ist. Der heterocyclische 5-Ring weist ein asymmetrisches Zentrum auf. Insbesondere für eine Gruppe dieser Wirkstoffe, die einen 2,5-Dioxoimidazolidinring, also einen Hydantoinring, enthalten, der über eine 1,4-Phenyleneinheit mit der Amino- oder Aminomethyl-, Amidino- oder Guanidinogruppierung verknüpft ist, hat es sich vor allem bei der Herstellung von Wirkstoffen mit einheitlicher Konfiguration am asymmetrischen Zentrum des 5-Ringes als vorteilhaft erwiesen, von den entsprechenden Hydantoinen der allgemeinen Formel I als Zwischenprodukten auszugehen.

Gegenstand der vorliegenden Erfindung sind daher Hydantoinderivate der allgemeinen Formel I, 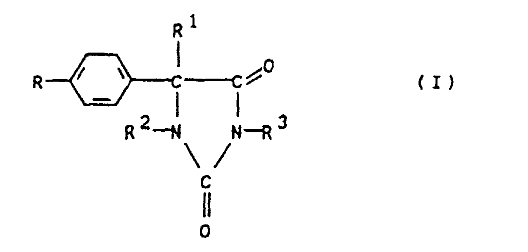 worin
- R: Cyano, C(=NH)-O-(C₁-C₆)-Alkyl, C(=NH)-NH-X, CH₂-NH-X oder NH-X¹ bedeutet;
- X: Wasserstoff, (C₁-C₆)-Alkyl, (C₁-C₆)-Alkylcarbonyl, (C₁-C₆)-Alkoxycarbonyl, (C₁-C₁₈)-Alkylcarbonyloxy(C₁-C₆)-alkoxycarbonyl, gegebenenfalls substituiertes (C₆-C₁₄)-Arylcarbonyl, gegebenenfalls substituiertes (C₆-C₁₄)-Aryloxycarbonyl, (C₆-C₁₄)-Aryl-(C₁-C₆)-alkoxycarbonyl, das im Arylrest auch substituiert sein kann, (R⁵O)₂P(O), Cyano, Hydroxy, (C₁-C₆)-Alkoxy, (C₆-C₁₄)-Aryl-(C₁-C₆)-alkoxy, das im Arylrest auch substituiert sein kann, oder Amino bedeutet;
- X¹: eine der Bedeutungen von X hat oder R'-NH-C(=N-R") bedeutet, wobei R' und R" unabhängig voneinander die Bedeutungen von X haben;
- R¹: Methyl, Ethyl, Propyl, Butyl, Pentyl, Hexyl, Isopropyl, Isobutyl, Isopentyl, Neopentyl, Isohexyl, 3-Methylpentyl, sec-Butyl, tert-Butyl, tert-Pentyl, im Arylrest gegebenenfalls substituiertes (C₆-C₁₄)-Aryl-(C₁-C₈)-alkyl oder (C₃-C₈)-Cycloalkyl bedeutet;
- R²: Wasserstoff, (C₁-C₈)-Alkyl, gegebenenfalls substituiertes (C₆-C₁₄)-Aryl, im Arylrest gegebenenfalls substituiertes (C₆-C₁₄)-Aryl-(C₁-C₈)-alkyl oder (C₃-C₈)-Cycloalkyl bedeutet;
- R³: Wasserstoff oder CH₂-CO-OR⁴ bedeutet;
- R⁴: Wasserstoff, (C₁-C₆)-Alkyl, im Arylrest gegebenenfalls substituiertes (C₆-C₁₄)-Aryl-(C₁-C₆)-alkyl oder gegebenenfalls substituiertes (C₆-C₁₄)-Aryl bedeutet;
- R⁵: Wasserstoff, (C₁-C₈)-Alkyl, gegebenenfalls substituiertes (C₆-C₁₄)-Aryl oder (C₆-C₁₄)-Aryl-(C₁-C₈)-alkyl, das im Arylrest auch substituiert sein kann, bedeutet;
wobei aber, wenn R³ für Wasserstoff steht, R nicht für CN, NH₂ oder CH₂-NH₂ stehen kann;
und wobei, wenn die Verbindungen der allgemeinen Formel I hinsichtlich des asymmetrischen Zentrums im Hydantoinring in racemischer Form vorliegen und gleichzeitig R³ für Methoxycarbonylmethyl, R¹ für Methyl und R² für Wasserstoff oder Methyl steht, R nicht für CN oder C(=NH)-OC₂H₅ stehen kann;
und wobei, wenn die Verbindungen der allgemeinen Formel I hinsichtlich des asymmetrischen Zentrums im Hydantoinring in racemischer Form vorliegen und gleichzeitig R³ für Methoxycarbonylmethyl oder Hydroxycarbonylmethyl, R¹ für Methyl und R² für Wasserstoff oder Methyl steht, R nicht für NH₂, CH₂-NH₂, C(=NH)-NH₂, tert-Butyloxycarbonylaminomethyl oder Benzyloxycarbonylguanidino stehen kann;
und wobei, wenn die Verbindungen der allgemeinen Formel I hinsichtlich des asymmetrischen Zentrums im Hydantoinring in racemischer Form vorliegen und gleichzeitig R¹ für Methyl und R² und R³ für Wasserstoff steht, R nicht für NH-CO-CH₃ stehen kann;
und wobei eine Arylgruppe, die substituiert ist, einen, zwei oder drei gleiche oder verschiedene Substituenten aus der Reihe (C₁-C₈)-Alkyl, (C₁-C₈)-Alkoxy, Halogen, Nitro, Amino, Trifluormethyl, Hydroxy, Methylendioxy, Cyano, Hydroxycarbonyl, Aminocarbonyl, (C₁-C₄)-Alkoxycarbonyl, Phenyl, Phenoxy, Benzyloxy, (R⁵O)₂P(O), (R⁵O)₂P(O)-O-, Tetrazolyl trägt;
und ihre Salze.

Gegenstand der vorliegenden Erfindung sind alle stereoisomeren Formen der Verbindungen der allgemeinen Formel I. Umfaßt werden also insbesondere, wenn neben dem asymmetrischen Zentrum im Hydantoinring keine weiteren optisch aktiven Zentren vorliegen, die Enantiomeren mit R-Konfiguration und die mit S-Konfiguration am Hydantoin-C-Atom und neben den reinen Enantiomeren auch das Racemat sowie Gemische der Enantiomeren in beliebigen Mengenverhältnissen. Liegen neben dem optisch aktiven C-Atom im Hydantoinring weitere asymmetrische Zentren vor, z.B. in Alkyl-, Cycloalkyl- oder Arylalkylresten, so können die optisch aktiven Atome im Molekül unabhängig voneinander R- oder S-Konfiguration haben, und an jedem dieser Zentren kann unabhängig von den anderen eine einheitliche Konfiguration vorliegen oder es kann ein Gemisch der konfigurationsisomeren Formen im Verhältnis 1:1 oder in beliebigem Mengenverhältnis vorliegen. Die vorliegende Erfindung umfaßt also sowohl alle reinen Enantiomeren und Enantiomerengemische als auch alle Diastereomeren und Diastereomerengemische. Sie umfaßt auch Salze der Verbindungen der allgemeinen Formel I mit optisch aktiven Säuren oder Basen.

Die Verbindungen der allgemeinen Formel I können bewegliche Wasserstoffatome enthalten, also in verschiedenen tautomeren Formen vorliegen. Auch alle diese Tautomeren sind Gegenstand der vorliegenden Erfindung.

Cycloalkylreste sind insbesondere Cyclopropyl, Cyclobutyl, Cyclopentyl, Cyclohexyl, Cycloheptyl und Cyclooctyl, die aber auch durch beispielsweise (C₁-C₄)-Alkyl substituiert sein können. Beispiele für substituierte Cycloalkylreste sind 4-Methylcyclohexyl und 2,3-Dimethylcyclopentyl.

Alkylreste können geradkettig oder verzweigt sein. Dies gilt auch, wenn sie Substituenten tragen oder als Substituenten anderer Reste auftreten, beispielsweise in Alkylcarbonyl-, Alkoxy-, Alkoxycarbonyl- oder Aralkylresten.

Beispiele für geeignete C₁-C₁₈-Alkylreste sind: Methyl, Ethyl, Propyl, Butyl, Pentyl, Hexyl, Heptyl, Octyl, Decyl, Undecyl, Dodecyl, Tridecyl, Pentadecyl, Hexadecyl, Heptadecyl, Octadecyl, Isopropyl, Isobutyl, Isopentyl, Neopentyl, Isohexyl, 3-Methylpentyl, 2,3,5-Trimethylhexyl, sec.-Butyl, tert.-Butyl, tert.-Pentyl. Bevorzugte Alkylreste sind Methyl, Ethyl, Propyl, Isopropyl, Butyl, Isobutyl, sec.-Butyl und tert.-Butyl.

(C₆-C₁₄)-Arylgruppen sind beispielsweise Phenyl, Naphthyl, Biphenylyl oder Fluorenyl, wobei 1-Naphthyl, 2-Naphthyl und insbesondere Phenyl bevorzugt sind. Arylreste, insbesondere Phenylreste, können unsubstituiert sein oder wie angegeben einfach, zweifach oder dreifach substituiert sein durch gleiche oder verschiedene Reste aus der Reihe (C₁-C₈)-Alkyl, insbesondere (C₁-C₄)-Alkyl, (C₁-C₈)-Alkoxy, insbesondere (C₁-C₄)-Alkoxy, Halogen, Nitro, Amino, Trifluormethyl, Hydroxy, Methylendioxy, Cyan, Hydroxycarbonyl, Aminocarbonyl, (C₁-C₄)-Alkoxycarbonyl, Phenyl, Phenoxy, Benzyloxy, (R⁵O)₂P(O), (R⁵O)₂P(O)-O-, Tetrazolyl. Entsprechendes gilt beispielsweise für Reste wie Aralkyl oder Arylcarbonyl. Aralkylreste sind insbesondere Benzyl sowie 1- und 2-Naphthylmethyl und 9-Fluorenylmethyl, die auch substituiert sein können. Substituierte Aralkylreste sind beispielsweise Halobenzyl oder (C₁-C₄)-Alkoxybenzyl.

In monosubstituierten Phenylresten kann sich der Substituent in der 2-, der 3- oder der 4-Position befinden, wobei die 3- und die 4-Position bevorzugt sind. Ist Phenyl zweifach substituiert, können die Substituenten in 1,2-, 1,3- oder 1,4-Position zueinander stehen. Bevorzugt sind in zweifach substituierten Phenylresten die beiden Substituenten in der 3- und der 4- oder der 3- und der 5-Position, bezogen auf die Verknüpfungsstelle, angeordnet.

Halogen steht für Fluor, Chlor, Brom oder Jod, insbesondere für Fluor oder Chlor.

Funktionelle Gruppen in den Verbindungen der allgemeinen Formel I können geschützt vorliegen. Geeignete Schutzgruppen wie z.B. Urethanschutzgruppen oder Carboxylschutzgruppen sind bei Hubbuch, Kontakte (Merck) 1979, Nr. 3, Seiten 14 bis 23 und bei Büllesbach, Kontakte (Merck) 1980, Nr. 1, Seiten 23 bis 35 beschrieben. Insbesondere seien genannt: Aloc, Pyoc, Fmoc, Tcboc, Z, Boc, Ddz, Bpoc, Adoc, Msc, Moc, Z(NO₂), Z(Halₙ), Bobz, Iboc, Adpoc, Mboc, Acm, tert.-Butyl, OBzl, ONbzl, OMbzl, Bzl, Mob, Pic, Trt.

Salze der Verbindungen der allgemeinen Formel I können Vorteile bei der Herstellung bzw. Isolierung der Verbindungen der allgemeinen Formel I, ihrer Lagerung oder der Folgeumsetzung aufweisen. Solche Salze können beispielsweise bei Verbindungen der allgemeinen Formel I, welche saure Gruppen, z.B. Carboxy, enthalten, Alkali- oder Erdalkalimetallsalze sein, wie z.B. Lithium-, Natrium-, Kalium-, Magnesium- oder Calciumsalze, sowie Salze mit Ammoniak oder organischen Aminen, wie z.B. Triethylamin, Ethyldiisopropylamin, N-Ethylmorpholin, Pyridin oder optisch aktiven Basen wie z.B. 1-Phenylethylamin.

Verbindungen der allgemeinen Formel I, welche basische Gruppen, z.B. eine Aminogruppe, eine Amidinogruppe oder eine Guanidinogruppe enthalten, können mit anorganischen Säuren, wie z.B. Salzsäure, Bromwasserstoffsäure, Iodwasserstoffsäure, Tetrafluoroborsäure, Schwefelsäure oder Phosphorsäure, und mit organischen Carbon- oder Sulfonsäuren, wie z.B. Essigsäure, Citronensäure, Benzoesäure, Maleinsäure, Fumarsäure, Weinsäure und anderen optisch aktiven Säuren, Methansulfonsäure, Naphthalinsulfonsäuren oder p-Toluolsulfonsäure, Salze bilden.

R steht bevorzugt für Cyano oder C(=NH)-NH-X.

X steht bevorzugt für Wasserstoff, (C₁-C₆)-Alkylcarbonyl, (C₁-C₆)-Alkoxycarbonyl oder im Arylrest gegebenenfalls substituiertes (C₆-C₁₄)-Aryl-(C₁-C₆)-alkoxycarbonyl, besonders bevorzugt für Wasserstoff.

R¹ steht bevorzugt für Methyl, Ethyl, Propyl, Butyl, Pentyl, Hexyl, Isopropyl, Isobutyl, Isopentyl, Neopentyl, Isohexyl, 3-Methylpentyl, sec-Butyl, tert-Butyl, tert-Pentyl, im Phenylrest gegebenenfalls substituiertes Phenyl-(C₁-C₈)-alkyl oder (C₃-C₈)-Cycloalkyl, besonders bevorzugt für (C₁-C₄)-Alkyl, Cyclopropyl oder Benzyl, ganz besonders bevorzugt für Methyl.

R² steht bevorzugt für Wasserstoff, (C₁-C₈)-Alkyl, gegebenenfalls substituiertes Phenyl, im Phenylrest gegebenenfalls substituiertes Phenyl-(C₁-C₈)-alkyl oder (C₃-C₈)-Cycloalkyl, besonders bevorzugt für Wasserstoff oder (C₁-C₄)-Alkyl, ganz besonders bevorzugt für Wasserstoff.

R³ steht bevorzugt für CH₂-CO-OR⁴, besonders bevorzugt für CH₂-COOH.

R⁴ steht bevorzugt für Wasserstoff oder (C₁-C₄)-Alkyl.

Bevorzugt liegt am asymmetrischen Zentrum im Hydantoinring eine einheitliche Konfiguration vor, besonders bevorzugt die S-Konfiguration.

Bevorzugte Verbindungen der allgemeinen Formel I sind solche, in denen einer oder mehrere Substituenten bevorzugte Bedeutungen haben. Besonders bevorzugt sind Verbindungen der allgemeinen Formel I, worin
- R: für Cyano oder C(=NH)-NH-X steht;
- X: für Wasserstoff, (C₁-C₆)-Alkylcarbonyl, (C₁-C₆)-Alkoxycarbonyl oder gegebenenfalls substituiertes (C₆-C₁₄)-Aryl-(C₁-C₆)-alkoxycarbonyl steht;
- R¹: für Methyl, Ethyl, Propyl, Butyl, Pentyl, Hexyl, Isopropyl, Isobutyl, Isopentyl, Neopentyl, Isohexyl, 3-Methylpentyl, sec-Butyl, tert-Butyl, tert-Pentyl, im Arylrest gegebenenfalls substituiertes (C₆-C₁₄)-Aryl-(C₁-C₈)-alkyl oder (C₃-C₈)-Cycloalkyl steht;
- R²: für Wasserstoff, (C₁-C₈)-Alkyl, gegebenenfalls substituiertes Phenyl, im Phenylrest gegebenenfalls substituiertes Phenyl-(C₁-C₈)-alkyl oder (C₃-C₈)-Cycloalkyl steht;
- R³: für CH₂-CO-OR⁴ steht;
- R⁴: für Wasserstoff, (C₁-C₆)-Alkyl, im Arylrest gegebenenfalls substituiertes (C₆-C₁₄)-Aryl-(C₁-C₆)-alkyl oder gegebenenfalls substituiertes (C₆-C₁₄)-Aryl steht;
wobei aber, wenn die Verbindungen der allgemeinen Formel I hinsichtlich des asymmetrischen Zentrums im Hydantoinring in racemischer Form vorliegen und gleichzeitig R³ für Methoxycarbonylmethyl, R¹ für Methyl und R² für Wasserstoff oder Methyl steht, R nicht für CN stehen kann;
und wobei, wenn die Verbindungen der allgemeinen Formel I hinsichtlich des asymmetrischen Zentrums im Hydantoinring in racemischer Form vorliegen und gleichzeitig R³ für Methoxycarbonylmethyl oder Hydroxycarbonylmethyl, R¹ für Methyl und R² für Wasserstoff oder Methyl steht, R nicht für C(=NH)-NH₂ stehen kann.

Ganz besonders bevorzugt sind Verbindungen der allgemeinen Formel I, worin
- R: für Cyano oder C(=NH)-NH₂ steht;
- R¹: für (C₁-C₄)-Alkyl, Cyclopropyl oder Benzyl, insbesondere Methyl, steht;
- R²: für Wasserstoff oder (C₁-C₄)-Alkyl, insbesondere Wasserstoff, steht;
- R³: für CH₂-COOH oder CH₂-COO-(C₁-C₄)-Alkyl, insbesondere CH₂-COOH, steht;
wobei aber, wenn die Verbindungen der allgemeinen Formel I hinsichtlich des asymmetrischen Zentrums im Hydantoinring in racemischer Form vorliegen und gleichzeitig R³ für Methoxycarbonylmethyl, R¹ für Methyl und R² für Wasserstoff oder Methyl steht, R nicht für CN stehen kann;
und wobei, wenn die Verbindungen der allgemeinen Formel I hinsichtlich des asymmetrischen Zentrums im Hydantoinring in racemischer Form vorliegen und gleichzeitig R³ für Methoxycarbonylmethyl oder Hydroxycarbonylmethyl, R¹ für Methyl und R² für Wasserstoff oder Methyl steht, R nicht für C(=NH)-NH₂ stehen kann.

Darüberhinaus bevorzugt sind Verbindungen der allgemeinen Formel I, die hinsichtlich des asymmetrischen Zentrums im Hydantoinring in enantiomerenreiner Form vorliegen, insbesondere die Isomeren mit der S-Konfiguration.

Auch bei allen bevorzugten Verbindungen der allgemeinen Formel I sind natürlich die Salze ebenfalls Gegenstand der vorliegenden Erfindung.

Die erfindungsgemäßen Verbindungen der allgemeinen Formel I können auf verschiedenen Wegen hergestellt werden, beispielsweise den im folgenden erläuterten Wegen A, B und C, wobei die einzelnen Schritte vielfach nach oder analog zu literaturbekannten Vorschriften durchgeführt werden können.

### Weg A:

Verbindungen der allgemeinen Formel I können hergestellt werden, indem man Verbindungen der allgemeinen Formel II, 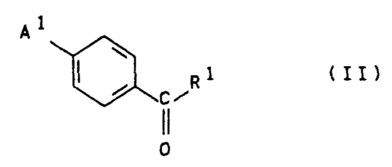 in der A¹ für Halogen, bevorzugt Brom, oder Nitro steht und R¹ die oben angegebenen Bedeutungen hat, unter den bekannten Bedingungen der Bucherer-Reaktion zu Verbindungen der allgemeinen Formel III, 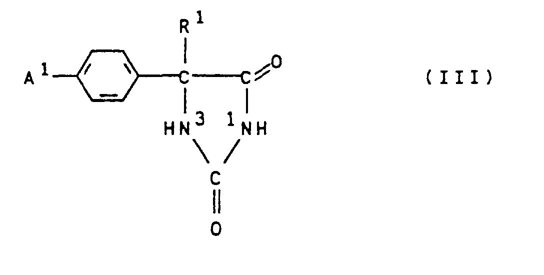 umsetzt, aus denen durch Hydrolyse des Hydantoins, z.B. mit Natronlauge in Anlehnung an das von H.T. Bucherer und V.A. Lieb, J. Prakt. Chem. 141 (1934), 5, beschriebene Verfahren, die Aminosäuren der allgemeinen Formel IV 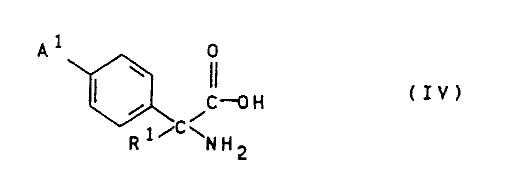 erhalten werden können. Aus diesen sind nach bekannten Verfahren (siehe z.B. L. Birkhofer und R. Modic, Liebigs Ann. Chem. 628 (1959), 168) die Aminosäureester der allgemeinen Formel V, 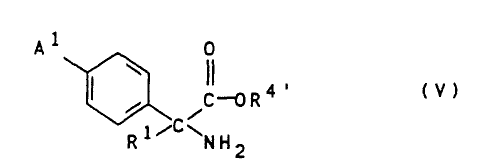 erhältlich, in der R^{4'} die oben für R⁴ angegebenen Bedeutungen mit Ausnahme von Wasserstoff haben kann. Diese können mit Isocyanatoessigsäureestern der allgemeinen Formel VI,

O=C=N-CH₂-COOR^{4"} (VI)

in der R^{4"} ebenfalls die oben für R⁴ angegebenen Bedeutungen mit Ausnahme von Wasserstoff haben kann, zu den Verbindungen der allgemeinen Formel VII 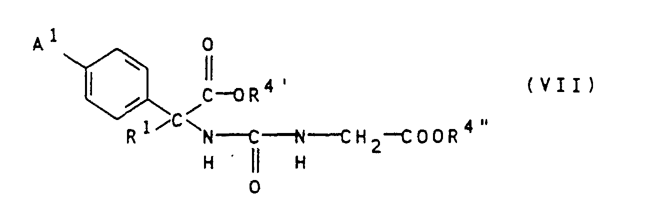 umgesetzt werden, die sich unter sauren Bedingungen, z.B. in wäßriger Säure, zu den Hydantoinessigsäuren der allgemeinen Formel VIII 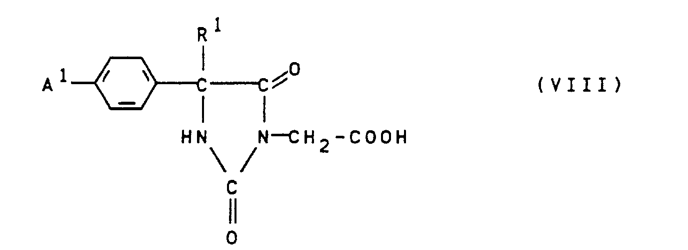 cyclisieren lassen.
Verbindungen der allgemeinen Formel VIII, in der A¹ für Halogen steht, können nach an sich bekannten Verfahren (siehe z.B. G.P. Ellis und T.M. Romney-Alexander, Chem. Rev. 87 (1987), 779-794), z.B. durch einen Brom-Cyan-Austausch, in Verbindungen der allgemeinen Formel Ia 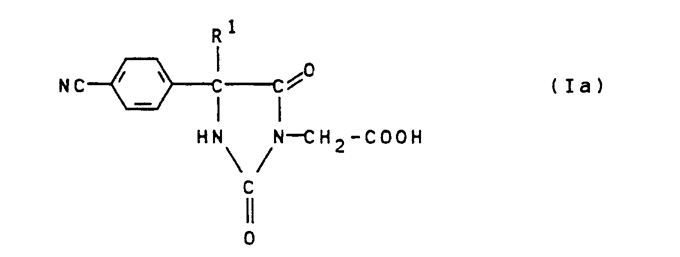 umgewandelt werden. Verbindungen der allgemeinen Formel I, in denen R die Bedeutung C(=NH)-O-(C₁-C₆)-Alkyl hat, sind aus den Verbindungen der allgemeinen Formal Ia durch Anlagerung der (C₁-C₆)-Alkanole im sauren wasserfreien Medium, z.B. in Dioxan oder im wasserfreien reinen Alkohol, erhältlich. Anschließende Aminolyse der Imidsäureester, z.B. durch Behandlung mit Ammoniak in Alkoholen, wie Methanol, Ethanol oder Isopropanol (siehe z.B. G. Wagner, P. Richter und C. Grabe, Pharmazie 29 (1974), 12-15), führt zu Amidinen, also Verbindungen der allgemeinen Formel I, in denen R die Bedeutung C(=NH)NH₂ hat. Eine weitere Methode, Amidine herzustellen, ist die Anlagerung von Schwefelwasserstoff an die Cyanogruppe der Verbindungen der allgemeinen Formel Ia, gefolgt von einer Methylierung des entstandenen Thioamids und anschließender Umsetzung z.B. mit Ammoniak (vgl. DDR-Patent Nr. 235 866). Verbindungen der allgemeinen Formel I, in denen R die Bedeutung CH₂NH₂ hat, können aus den Verbindungen der allgemeinen Formel Ia durch Reduktion der Cyanogruppe nach an sich bekannten Verfahren hergestellt werden.

Verbindungen der allgemeinen Formel VIII, in der A¹ für NO₂ steht, können nach an sich bekannten Verfahren durch Reduktion der Nitrogruppe in Verbindungen der allgemeinen Formel I umgewandelt werden, in denen R die Bedeutung NH₂ hat. Aus dieser Aminofunktion kann mit den folgenden Reagentien eine Guanidinogruppierung erhalten werden:
1. O-Methylisoharnstoff
   (S. Weiss und H. Krommer, Chemiker-Zeitung 98 (1974), 617 - 618),
2. S-Methylisothioharnstoff
   R.F. Borne, M.L. Forrester und I.W. Waters, J. Med. Chem. 20 (1977), 771 - 776),
3. Nitro-S-methylisothioharnstoff
   (L.S. Hafner und R. Evans, J. Org. Chem. 24 (1959), 1157),
4. Formamidinsulfonsäure
   (K. Kim, Y.-T. Lin und H.S. Mosher, Tetrahedron Lett. 29 (1988), 3183 - 3186),
5. 3,5-Dimethyl-1-pyrazolyl-formamidinium-nitrat
   (F.L. Scott, D.G. O'Donovan und J. Reilly, J. Amer. Chem. Soc. 75 (1953), 4053 - 4054),
6. N,N'-Di-tert.-butyloxycarbonyl-S-methyl-isothioharnstoff
   (R.J. Bergeron und J.S. McManis, J. Org. Chem. 52 (1987), 1700 - 1703),
7. N-Alkoxycarbonyl-, N,N'-Dialkoxycarbonyl-, N-Alkylcarbonyl- und N,N'-Dialkylcarbonyl-S-methyl-isothioharnstoff (H. Wollweber, H. Kölling, E. Niemers, A. Widding, P. Andrews, H.-P. Schulz und H. Thomas, Arzneim. Forsch./Drug Res. 34 (1984), 531 - 542).

Verbindungen der allgemeinen Formel I, in denen R³ für CH₂-COOR⁴ steht und R⁴ eine andere Bedeutung als Wasserstoff hat, lassen sich aus den Verbindungen der allgemeinen Formel VIII durch Veresterung der Carboxylgruppe nach an sich bekannten Verfahren und anschließende Transformation des Restes A¹ in den Rest R wie vorstehend beschrieben erhalten. Verbindungen der allgemeinen Formel I, in denen R² eine andere Bedeutung als Wasserstoff hat, sind aus den an der Carboxylgruppe veresterten Verbindungen der allgemeinen Formel VIII durch Alkylierung, Cycloalkylierung, Arylierung bzw. Aralkylierung an der NH-Gruppe des Hydantoins nach an sich bekannten Methoden und anschließende Transformation des Restes A¹ in den Rest R wie vorstehend beschrieben erhältlich. Verbindungen der allgemeinen Formel I, in denen R² eine andere Bedeutung als Wasserstoff hat und R³ die Bedeutung CH₂-COOH hat, werden aus den Carbonsäureestern durch neuerliche Hydrolyse erhalten.

Verbindungen der allgemeinen Formel I, in denen R³ die Bedeutung Wasserstoff hat, können, wenn R² die Bedeutung Wasserstoff hat, aus den Verbindungen der allgemeinen Formel III durch die vorstehend beschriebenen Transformationen des Restes A¹ in den Rest R erhalten werden. Verbindungen der allgemeinen Formel I, in denen R³ die Bedeutung Wasserstoff und R² eine andere Bedeutung als Wasserstoff hat, können aus den Verbindungen der allgemeinen Formel III durch Schützen der N¹H-Funktion, Alkylierung, Cycloalkylierung, Arylierung bzw. Aralkylierung der freien N³H-Gruppe, Freisetzen der N¹H-Funktion und Transformation des Restes A¹ in den Rest R erhalten werden. Sollen enantiomerenreine Verbindungen der allgemeinen Formel I, in denen R³ die Bedeutung Wasserstoff hat, hergestellt werden und wird die Enantiomerentrennung auf der Stufe der Verbindungen der allgemeinen Formel V durchgeführt, so können die Verbindungen der allgemeinen Formel V nach literaturbekannten Verfahren, z.B. nach M.J.O. Anteunis et al., Bull. Soc. Chim. Belg. 96 (1987), 459, wieder zu den Hydantoinen der allgemeinen Formel III umgesetzt werden, mit denen dann die genannten Folgereaktionen durchgeführt werden.

Verbindungen der allgemeinen Formel I, in denen X oder X¹ eine andere Bedeutung als Wasserstoff haben, sind aus oder analog zu den in der Amino-, Amidino- und Guanidinogruppe unsubstituierten Verbindungen nach Standardverfahren erhältlich, beispielsweise durch Acylierung, oder Alkylierung der unsubstituierten Verbindungen oder indem Verbindungen der allgemeinen Formel VIII z.B. mit Hydroxylamin statt Ammoniak umgesetzt werden.

Verbindungen der allgemeinen Formel I, die hinsichtlich des asymmetrischen Zentrums im Hydantoinring enantiomerenrein sind, können z.B. durch Racematspaltung der Verbindungen der allgemeinen Formel V, beispielsweise durch Kristallisation der Salze mit R-Mandelsäure bzw. S-Mandelsäure, und Umwandlung der erhaltenen enantiomerenreinen Verbindungen der allgemeinen Formel V in solche der allgemeinen Formel I auf den erläuterten Wegen hergestellt werden. Zur Racematspaltung sind auch andere chirale Säuren geeignet. Bevorzugt werden leicht zugängliche oder käufliche optisch reine Verbindungen eingesetzt, wie Äpfelsäure, Weinsäure, Milchsäure, Camphersulfonsäure, Ketopinsäure (vgl. z.B. Organic Syntheses, Band 45, S. 55) oder Menthoxyessigsäure. Auch optisch reine Basen eignen sich zur Racematspaltung, wenn diese nicht auf der Stufe der Verbindungen der allgemeinen Formel V, sondern auf der Stufe der Aminosäuren der allgemeinen Formel IV durchgeführt wird und die Aminogruppe in einer reversibel geschützten Form vorliegt. Geeignete optisch reine Basen sind beispielsweise 1-Phenylethylamin, Ephedrin, Brucin, Strychnin oder Chinin. Auch enantiomerenreine Aminosäurederivate, bei denen entweder die Aminogruppe oder die Carbonsäuregruppe blockiert ist, eignen sich als chirale Hilfsreagenzien für die Herstellung von enantiomerenreinen Verbindungen der allgemeinen Formel I. Als geeignete Aminosäurederivate seien Pyroglutaminsäure, N-Acetylprolin, N-Formylthiazolidincarbonsäure, Phenylglycin-tert-butylester oder Valin-tert-butylester erwähnt.

Bei den Reaktionen zur Herstellung der Verbindungen der allgemeinen Formel I kann es angebracht sein, funktionelle Gruppen zeitweise durch geeignete übliche Schutzgruppenl zu blockieren, die nach an sich bekannten Methoden eingeführt und wieder entfernt werden könnedn (siehe z.B. oben angegebene Literatur). Z.B. können Guanidinogruppen durch NO₂- oder Mtr-Schutzgruppen blockiert werden. Aminogruppen, die nicht an der Reaktion beteiligt sind, können z.B. in geschützter Form als Boc- oder Z-Derivate vorliegen. Ein Aminogruppenschutz erübrigt sich, wenn die zu generierenden Aminogruppen noch als Nitro- oder Cyanogruppen vorliegen und erst nach der Reaktion, z.B. durch Hydrierung, erzeugt werden. Nicht an der Reaktion beteiligte Carboxygruppen werden bevorzugt als (C₁-C₆)-Alkyl-, insbesondere tert-Butyl-, oder als Benzylester geschützt. Nach der Reaktion werden die vorhandenen Schutzgruppen in geeigneter Weise abgespalten, beispielsweise Nitrogruppen (Guanidinoschutz), Benzyloxycarbonylgruppen und Benzylester abhydriert, Schutzgruppen vom tert-Butyltyp sauer gespalten oder 9-Fluorenylmethyloxycarbonylreste durch sekundäre Amine entfernt.

### Weg B:

Verbindungen der allgemeinen Formel I können auch hergestellt werden, indem man Verbindungen der allgemeinen Formel IX, 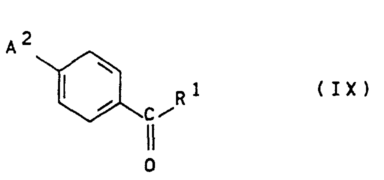 in der A² für Halogen, bevorzugt Brom, Nitro oder Cyano steht und R¹ die oben angegebenen Bedeutungen hat, unter den bekannten Bedingungen der Strecker-Reaktion zu den Aminonitrilen der allgemeinen Formel X 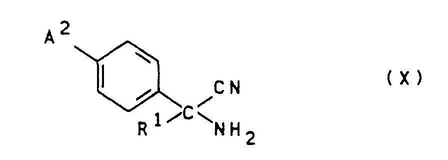 umsetzt, aus denen man nach Hydrolyse der aliphatischen Nitrilgruppe in Anlehnung an literaturbekannte Verfahren (siehe z.B. D. Döpp und H. Döpp in Methoden der organischen Chemie (Houben-Weyl), Thieme-Verlag, Stuttgart 1985, 4. Auflage, Band E 5, S. 1024 ff., oder F. Becke, H. Fleig und P. Päßler, Liebigs Ann. Chem. 749 (1971), 198), z.B. mit konzentrierten Mineralsäuren, wie konz. Salzsäure oder konz. Schwefelsäure, oder einem Gemisch aus Salzsäure und Ameisensäure, die Carbonsäureamide der allgemeinen Formel XI 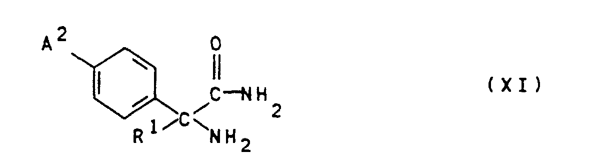 erhält. Diese können, wie unter Weg A erläutert, mit Isocyanatoessigsäureestern der allgemeinen Formel VI zu Verbindungen der allgemeinen Formel XII 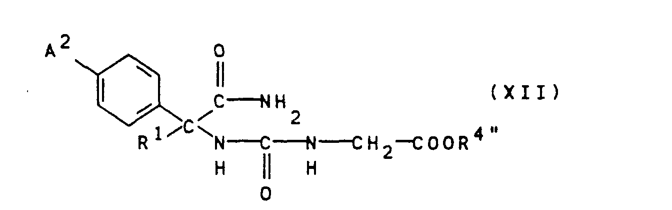 umgesetzt werden, in der R^{4"} die unter Weg A angegebenen Bedeutungen hat und aus denen man durch Cyclisierung analog den Angaben unter Weg A Verbindungen der allgemeinen Formel XIII 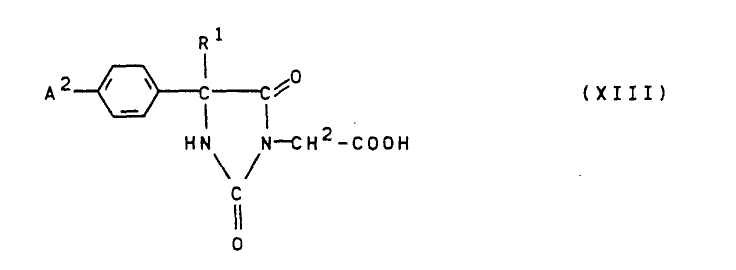 erhält, die wie unter A beschrieben in Verbindungen der allgemeinen Formel I umgewandelt werden können.

Verbindungen der allgemeinen Formel I, in denen R³ die Bedeutung Wasserstoff hat, können hergestellt werden, indem man die Verbindungen der allgemeinen Formel XI analog literaturbekannten Verfahren, z.B. wie in der EP-A-173522 beschrieben mit Dimethylcarbonat oder durch Benzyloxycarbonylierung der Aminogruppe in der 2-Position und anschließende Behandlung mit einer Base, zu den Hydantoinen der allgemeinen Formel XIV 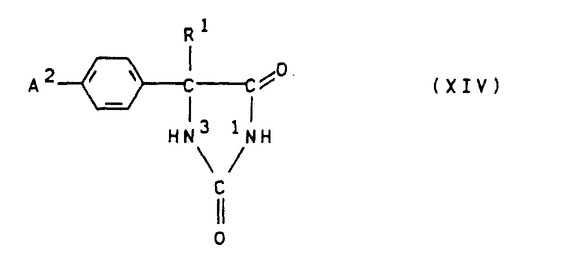 cyclisiert. Gewünschtenfalls können dann, analog den Angaben unter Weg A, durch Schützen der N¹H-Funktion, Alkylierung, Cycloalkylierung, Arylierung bzw. Aralkylierung der N³H-Gruppe und Freisetzen der N¹H-Funktion Substituenten in der 3-Position des Hydantoins eingeführt werden und/oder, analog den Angaben unter Weg A, durch Transformation des Restes A² in den Rest R Verbindungen der allgemeinen Formel I erhalten werden.

Im übrigen gelten hier die unter Weg A gegebenen Erläuterungen, beispielsweise zur Einführung von Substituenten X an den Amino-, Amidino- und Guanidinogruppen oder zur Verwendung von Schutzgruppen bei den Reaktionsschritten, entsprechend. Verbindungen der allgemeinen Formel I, die hinsichtlich des asymmetrischen Zentrums im Hydantoinring enantiomerenrein sind, können auf dem Weg B z.B. durch Racematspaltung der Verbindungen der allgemeinen Formel XI, beispielsweise durch Kristallisation der Salze mit D-Weinsäure bzw. L-Weinsäure, und Umwandlung der erhaltenen enantiomerenreinen Verbindungen der allgemeinen Formel XI in solche der allgemeinen Formel I auf den erläuterten Wegen hergestellt werden. Zur Racematspaltung sind auch andere chirale Säuren geeignet, beispielsweise die oben unter Weg A angegebenen, einschließlich der dort genannten Aminosäuren mit blockierter Aminogruppe.

### Weg C:

Zu Verbindungen der allgemeinen Formel I gelangt man auch, indem man Verbindungen der allgemeinen Formel IX, in der A² und R¹ die dort angegebenen Bedeutungen haben, entsprechend den Angaben unter Weg A unter den Bedingungen der Bucherer-Reaktion zu den Hydantoinen der allgemeinen Formel XIV 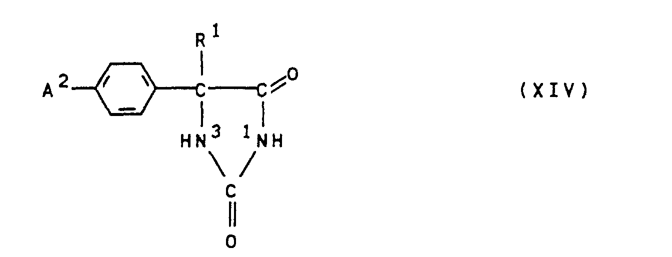 umsetzt und diese mit einem Halogenessigsäureester, beispielsweise Chloressigsäuremethylester, nach bekannten Verfahren an der N¹H-Funktion alkyliert. Die erhaltenen Verbindungen der allgemeinen Formel XV, 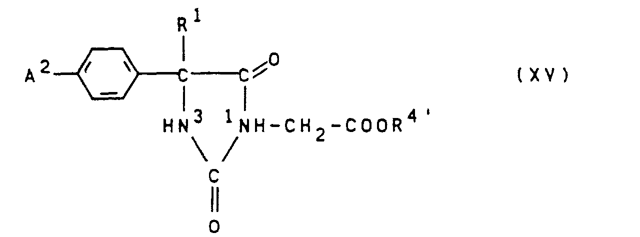 in der R^{4'} wie oben die für R⁴ angegebenen Bedeutungen mit Ausnahme von Wasserstoff haben kann, können entsprechend den obigen Angaben durch Einführung eines Alkyl-, Cycloalkyl-, Aryl- oder Aralkylsubstituenten an der N³H-Funktion und/oder Transformation des Restes A² in den Rest R und/oder Verseifung der Carbonestergruppe in die gewünschten Verbindungen der allgemeinen Formel I überführt werden.

Die Verbindungen der allgemeinen Formel I eignen sich in vorteilhafter Weise als Zwischenprodukte für die Herstellung der in den deutschen Patentanmeldungen P 43 48 944 und P 44 27 979 und der PCT-Anmeldung PCT/EP 94/03491 beschriebenen, die Zell-Zell-Adhäsion hemmenden pharmazeutischen Wirkstoffe, wenn diese als N-terminale Komponente einen 2,5-Dioxoimidazolidinring enthalten, der über eine 1,4-Phenyleneinheit mit einer substituierten oder unsubstituierten Amino- oder Aminomethyl-, Amidino- oder Guanidinogruppierung verknüpft ist, insbesondere wenn Wirkstoffe hergestellt werden sollen, die hinsichtlich des asymmetrischen Zentrums im Hydantoinring enantiomerenrein sein sollen. Eine Trennung der Isomeren kann auf verschiedene Stufen der Wirkstoffherstellung erfolgen, je nach der C-terminalen Komponente des Wirkstoffs kann es aber besonders günstig sein, die Verbindungen der allgemeinen Formel I in hinsichtlich des asymmetrischen Zentrums im Hydantoinring enantiomerenreiner Form in die Wirkstoffsynthese einzusetzen, damit z.B. von wertvollen C-terminalen Komponenten nicht die Hälfte der Substanz verlorengeht.

Die Verknüpfung der die N-terminale Komponente der Wirkstoffe darstellenden Verbindungen der allgemeinen Formel I mit einer weiteren Komponente des Wirkstoffmoleküls oder mit der kompletten C-terminalen Komponente kann nach üblichen Methoden erfolgen. Verbindungen der allgemeinen Formel I, in denen R³ die Bedeutung CH₂COOR⁴ hat, können z.B., wenn die Verbindung der allgemeinen Formel I über eine Aminogruppe der weiteren Komponente oder der kompletten C-terminalen Komponente verknüpft werden soll, nach dem Fachmann an sich bekannten Kupplungsmethoden der Peptidchemie (siehe z.B. Houben-Weyl, Methoden der organischen Chemie, Band 15/1 und 15/2, Stuttgart 1974) zur Folgestufe umgesetzt werden. Soll eine Verbindung der allgemeinen Formel I, in der R³ die Bedeutung CH₂COOH hat, mit einer Aminogruppe kondensiert werden, so erfolgt dabei in der Regel zunächst eine Aktivierung der Carbonsäuregruppierung, z.B. durch Überführung in ein Säurechlorid, in einen aktivierten Ester oder in ein gemischtes Anhydrid oder durch Umsetzung mit Kupplungsreagenzien wie Carbodiimiden, z.B. DCC (Dicyclohexylcarbodiimid), oder TOTU (O-(Cyan-ethoxy-carbonyl-methylen)amino)-N,N,-N',N'-tetramethyluronium-tetrafluoroborat), wobei es vorteilhaft sein kann, neben dem eigentlichen Aktivierungsmittel noch übliche Hilfsstoffe zuzugeben, bei der Aktivierung der Carbonsäuregruppierung mit DCC beispielsweise 1-Hydroxybenztriazol (HOBt). Bei diesen Verknüpfungsreaktionen kann es angebracht sein, funktionelle Gruppen zunächst durch Schutzgruppen zu schützen (siehe hierzu die obigen Erläuterungen zu Schutzgruppen).

Bezüglich der pharmazeutischen Wirkstoffe, für die die Verbindungen der allgemeinen Formel I als Zwischenprodukte dienen können, wird hier auf die deutschen Patentanmeldungen P 43 38 944 und P 44 27 979 und die PCT-Anmeldung PCT/EP94/03491 Bezug genommen. Beispielhaft genannt seien, ohne daß damit eine Einschränkung verbunden ist, als eine Gruppe dieser Wirkstoffe die Verbindungen der allgemeinen Formel XVI, 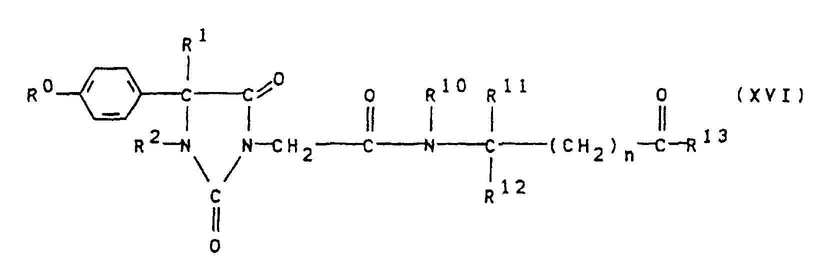 worin
- R⁰: C(=NH)-NH-X, CH₂-NH-X oder NH-X¹ bedeutet;
- X: Wasserstoff, (C₁-C₆)-Alkyl, (C₁-C₆)-Alkylcarbonyl, (C₁-C₆)-Alkaxycarbonyl, (C₁-C₁₈)-Alkylcarbonyloxy-(C₁-C₆)-alkoxycarbonyl, gegebenenfalls substituiertes (C₆-C₁₄)-Arylcarbonyl, gegebenenfalls substituiertes (C₆-C₁₄)-Aryloxycarbonyl, (C₆-C₁₄)-Aryl-(C₁-C₆)-alkoxycarbonyl, das im Arylrest auch substituiert sein kann, (R⁵O)₂P(O), Cyano, Hydroxy, (C₁-C₆)-Alkoxy, (C₆-C₁₄)-Aryl-(C₁-C₆)-alkoxy, das im Arylrest auch substituiert sein kann, oder Amino bedeutet;
- X¹: eine der Bedeutungen von X hat oder R'-NH-C(=N-R") bedeutet, wobei R' und R" unabhängig voneinander die Bedeutungen von X haben;
- R¹: Methyl, Ethyl, Propyl, Butyl, Pentyl, Hexyl, Isopropyl, Isobutyl, Isopentyl, Neopentyl, Isohexyl, 3-Methylpentyl, sec-Butyl, tert-Butyl, tert-Pentyl, im Arylrest gegebenenfalls substituiertes (C₆-C₁₄)-Aryl-(C₁-C₈)-alkyl oder (C₃-C₈)-Cycloalkyl bedeutet;
- R², R¹⁰ und R¹¹: unabhängig voneinander Wasserstoff, (C₁-C₈)-Alkyl, gegebenenfalls substituiertes (C₆-C₁₄)-Aryl, im Arylrest gegebenenfalls substituiertes (C₆-C₁₄)-Aryl-(C₁-C₈)-alkyl oder (C₃-C₈)-Cycloalkyl bedeuten;
- R⁵: Wasserstoff, (C₁-C₈)-Alkyl, gegebenenfalls substituiertes (C₆-C₁₄)-Aryl oder (C₆-C₁₄)-Aryl-(C₁-C₈)-alkyl, das im Arylrest auch substituiert sein kann, bedeutet;
- R¹²: Wasserstoff, (C₁-C₈)-Alkyl, gegebenenfalls substituiertes (C₆-C₁₄)-Aryl, im Arylrest gegebenenfalls substituiertes (C₆-C₁₄)-Asyl-(C₁-C₈)-alkyl, (C₃-C₈)-Cycloalkyl oder 2-, 3- oder 4-Pyridyl bedeutet;
- R¹³: Hydroxy, (C₁-C₁₈)-Alkoxy, (C₆-C₁₄)-Aryl-(C₁-C₈)-alkoxy, das im Arylrest auch substituiert sein kann, gegebenenfalls substituiertes (C₆-C₁₄)-Aryloxy, Amino oder Mono- oder Di-((C₁-C₁₈)-Alkyl)-amino bedeutet;
- n: eine ganze Zahl von 0 bis 6 bedeutet;
wobei eine Arylgruppe, die substituiert ist, einen, zwei oder drei gleiche oder verschiedene Substituenten aus der Reihe (C₁-C₈)-Alkyl, (C₁-C₈)-Alkoxy, Halogen, Nitro, Amino, Trifluormethyl, Hydroxy, Methylendioxy, Cyano, Hydroxycarbonyl, Aminocarbonyl, (C₁-C₄)-Alkoxycarbonyl, Phenyl, Phenoxy, Benzyloxy, (R⁵O)₂P(O), (R⁵O)₂P(O)-O-, Tetrazolyl trägt.

Bevorzugt werden unter Verwendung der Verbindungen der allgemeinen Formel I als Zwischenprodukte Wirkstoffe der allgemeinen Formel XVI hergestellt, in der einer oder mehrere der Reste bevorzugte Bedeutungen haben und/oder in der hinsichtlich des asymmetrischen Zentrums im Hydantoinring eine einheitliche Konfiguration, besonders bevorzugt die S-Konfiguration, vorliegt. Sind R¹¹ und R¹² verschieden, so stellt auch das diese Reste tragende Kohlenstoffatom ein asymmetrisches Zentrum dar. Bevorzugt ist es, wenn auch an diesen Zentrum eine einheitlich Konfiguration, besonders bevorzugt die S-Konfiguration, vorliegt. R⁰ steht bevorzugt für C(=NH)-NH-X, für die bevorzugten Bedeutungen der Reste X, R¹ und R² gelten auch hier die oben gemachten Angaben. R¹⁰ steht bevorzugt für Wasserstoff, (C₁-C₆)-Alkyl oder Benzyl, besonders bevorzugt für Wasserstoff. R¹¹ steht bevorzugt für Wasserstoff oder (C₁-C₈)-Alkyl, besonders bevorzugt für Wasserstoff. R¹² steht bevorzugt für gegebenenfalls substituiertes (C₆-C₁₄)-Aryl oder Pyridyl, besonders bevorzugt für gegebenenfalls substituiertes Phenyl, ganz besonders bevorzugt für unsubstituiertes Phenyl. R¹³ steht bevorzugt für Hydroxy oder (C₁-C₈)-Alkoxy, besonders bevorzugt für (C₁-C₄)-Alkoxy. n steht bevorzugt für eine ganze Zahl von 0 bis 3, besonders bevorzugt für die Zahlen 1 oder 2, ganz besonders bevorzugt für die Zahl 1.

Die Herstellung der beispielhaft genannten Wirkstoffe der allgemeinen Formel XVI aus den Verbindungen der allgemeinen Formel I, insbesondere denen, in denen R³ die Bedeutung CH₂COOH hat, kann entsprechend den obigen Erläuterungen erfolgen, indem diese nach den dem Fachmann an sich bekannten Kupplungsmethoden der Peptidchemie mit Verbindungen der allgemeinen Formel XVII, 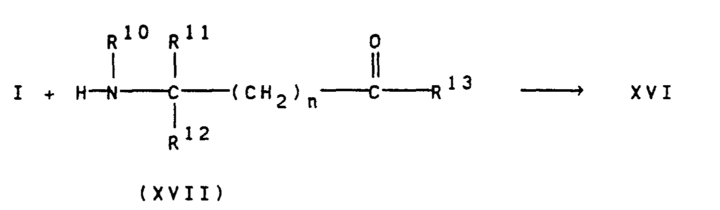 in der R¹⁰ bis R¹³ und n die für die Formel XVI angegebenen Bedeutungen haben, umgesetzt werden.

Die Herstellung der Wirkstoffe kann aber auch erfolgen, indem man zunächst - wie bereits erläutert - Verbindungen der allgemeinen Formel I, in denen R³ die Bedeutung Wasserstoff hat, mit einem Halogenessigsäureester umsetzt und das erhaltene Produkt oder die daraus hergestellte freie Säure mit den Verbindungen der allgemeinen Formel XVII kondensiert. Die Verbindungen der allgemeinen Formel I müssen nicht immer die direkten Vorstufen der angestrebten Wirkstoffe sein, sondern es können nach der Umsetzung mit den Verbindungen der allgemeinen Formel I noch ein oder mehrere weitere Reaktionsschritte auf dem Weg zum Wirkstoff folgen. Je nach der Struktur des C-terminalen Teils des Wirkstoffs kann es günstig sein, den Wirkstoff in mehreren Schritten vom N-terminalen Ende her, also gewissermaßen an der Verbindung der allgemeinen Formel I, aufzubauen.

Werden Verbindungen der allgemeinen Formel I, in denen R für eine unsubstituierte oder substituierte Amino- oder Aminomethyl-, Amidino- oder Guanidinogruppierung steht, mit der kompletten C-terminalen Komponente des Wirkstoffs verknüpft, so wird in einem Schritt aus den Verbindungen der allgemeinen Formel I der Wirkstoff erhalten. Es kann aber auch günstig sein, zunächst Verbindungen der allgemeinen Formel I, in denen R für Cyano steht, also Verbindungen der allgemeinen Formel Ib, 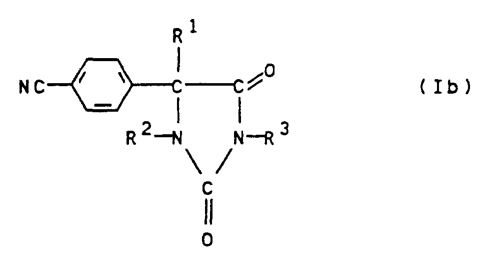 in der R¹, R² und R³ die oben angegebenen Bebedeutungen haben, mit der kompletten C-terminalen Komponente des Wirkstoffs zu verknüpfen und im letzten Schritt der Wirkstoffherstellung dann die Cyanogruppe nach den oben bereits erläuterten, an sich bekannten Verfahren in die Aminomethyl- oder Amidinogruppierung des Wirkstoffs zu überführen. Im Falle der beispielhaft genannten Wirkstoffe der allgemeinen Formel XVI, in denen R⁰ die Bedeutung C(=NH)-NH-X oder CH₂-NH-X hat, kann bei dieser Vorgehensweise z.B. eine Verbindung der allgemeinen Formel Ib, in der R³ die Bedeutung CH₂COOR⁴ hat, mit einer Verbindung der allgemeinen Formel XVII umgesetzt werden und das zunächst erhaltene Produkt dann durch Transformation der Cyanogruppe in eine Aminomethyl- oder Amidinogruppe in den Wirkstoff überführt werden.

Gegenstand der vorliegenden Erfindung sind auch die bei dieser Vorgehensweise erhaltenen, ebenfalls Zwischenprodukte oder auch direkte Vorstufen für die Wirkstoffe darstellenden Verbindungen der allgemeinen Formel XVIII, 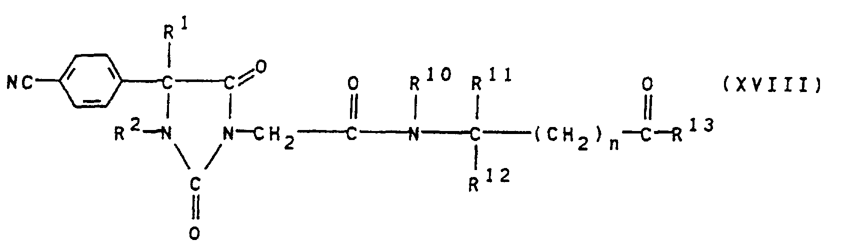 worin
- R¹: Methyl, Ethyl, Propyl, Butyl, Pentyl, Hexyl, Isopropyl, Isobutyl, Isopentyl, Neopentyl, Isohexyl, 3-Methylpentyl, sec-Butyl, tert-Butyl, tert-Pentyl, im Arylrest gegebenenfalls substituiertes (C₆-C₁₄)-Aryl-(C₁-C₈)-alkyl oder (C₃-C₈)-Cycloalkyl bedeutet;
- R², R¹⁰ und R¹¹: unabhängig voneinander Wasserstoff, (C₁-C₈)-Alkyl, gegebenenfalls substituiertes (C₆-C₁₄)-Aryl, im Arylrest gegebenenfalls substituiertes (C₆-C₁₄)-Aryl-(C₁-C₈)-alkyl oder (C₃-C₈)-Cycloalkyl bedeuten;
- R¹²: Wasserstoff, (C₁-C₈)-Alkyl, gegebenenfalls substituiertes (C₆-C₁₄)-Aryl, im Arylrest gegebenenfalls substituiertes (C₆-C₁₄₎-Aryl-(C₁-C₈)-alkyl, (C₃-C₈)-Cycloalkyl oder 2-, 3- oder 4-Pyridyl bedeutet;
- R¹³: Hydroxy, (C₁-C₁₈)-Alkoxy, (C₆-C₁₄)-Aryl-(C₁-C₈)-alkoxy, das im Arylrest auch substituiert sein kann, gegebenenfalls substituiertes (C₆-C₁₄)-Aryloxy, Amino oder Mono- oder Di-((C₁-C₁₈)-Alkyl)-amino bedeutet;
- n: eine ganze Zahl von 0 bis 6 bedeutet;
wobei eine Arylgruppe, die substituiert ist, einen, zwei oder drei gleiche oder verschiedene Substituenten aus der Reihe (C₁-C₈)-Alkyl, (C₁-C₈)-Alkoxy, Halogen, Nitro, Amino, Trifluormethyl, Hydroxy, Methylendioxy, Cyano, Hydroxycarbonyl, Aminocarbonyl, (C₁-C₄)-Alkoxycarbonyl, Phenyl, Phenoxy, Benzyloxy, (R⁵O)₂P(O), (R⁵O)₂P(O)-O-, Tetrazolyl trägt;
und ihre Salze.

Bezüglich der Alkyl- und Arylsubstituenten etc. gelten auch hier die obigen Erläuterungen. Auch hier werden sämtliche Stereoisomeren und sämtliche Mischungen von stereoisomeren Formen umfaßt. Die asymmetrischen Zentren im Hydantoinring und an dem die Gruppen R¹¹ und R¹² tragenden Kohlenstoffatom können unabhängig voneinander jeweils in der R-Konfiguration oder S-Konfiguration vorliegen.
Für die bevorzugten Bedeutungen der Substituenten in der allgemeinen Formel XVIII gelten wiederum die obigen Angaben. Bevorzugt liegt am asymmetrischen Zentrum im Hydantoinring als auch an dem die Gruppen R¹¹ und R¹² tragenden Kohlenstoffatom eine einheitliche Konfiguration vor, besonders bevorzugt an beiden die S-Konfiguration. Bevorzugt sind Verbindungen der allgemeinen Formel XVIII,
worin
R¹ für (C₁-C₄)-Alkyl, Cyclopropyl oder Benzyl, besonders bevorzugt für Methyl, steht;
R² für Wasserstoff oder (C₁-C₄)-Alkyl, besonders bevorzugt für Wasserstoff, steht;
R¹⁰ für Wasserstoff, (C₁-C₆)-Alkyl oder Benzyl, besonders bevorzugt für Wasserstoff, steht;
R¹¹ für Wasserstoff oder (C₁-C₈)-Alkyl, besonders bevorzugt für Wasserstoff, steht;
R¹² für gegebenenfalls substituiertes (C₆-C₁₄)-Aryl oder Pyridyl, besonders bevorzugt für gegebenenfalls substituiertes Phenyl, ganz besonders bevorzugt für unsubstituiertes Phenyl steht;
R¹³ für Hydroxy oder (C₁-C₈)-Alkoxy, besonders bevorzugt für (C₁-C₄)-Alkoxy steht;
n für eine ganze Zahl von 0 bis 3, besonders bevorzugt für die Zahlen 1 oder 2, ganz besonders bevorzugt für die Zahl 1 steht.

Darüber hinaus bevorzugt sind Verbindungen der allgemeinen Formel XVIII, in denen am asymmetrischen Zentrum im Hydantoinring als auch an dem die Reste R¹¹ und R¹² tragenden Kohlenstoffatom eine einheitliche Konfiguration vorliegt, insbesondere an beiden die S-Konfiguration.

Die Herstellung der Verbindungen der allgemeinen Formel XVIII kann, wie bereits erläutert, z.B. erfolgen, indem die Verbindungen der allgemeinen Formel Ib, in der R³ die Bedeutung CH₂-COOH hat, nach den üblichen, dem Fachmann an sich bekannten Kupplungsmethoden der Peptidchemie mit Verbindungen der allgemeinen Formel XVII gekuppelt werden, z.B. mit Hilfe von Reagenzien wie Carbodiimiden, z.B. DCC, oder z.B. mit TOTU. Die Umwandlung der Cyanogruppe in die in dem Wirkstoff mit zell-zell-adhäsionshemmender, insbesondere thrombocytenaggregationshemmender Wirkung enthaltene Gruppe, z.B. insbesondere in die Amidinogruppe und gewünschtenfalls deren durch den Rest X substituierte Derivate oder deren Salze, kann, wie ebenfalls bereits erläutert, ebenso nach den üblichen, dem Fachmann für diese Umwandlung bekannten Verfahren erfolgen, im Falle der Amidinogruppe beispielsweise durch Umsetzung der Cyanogruppe mit Hydroxyamin zur Amidoximgruppierung und anschließende Hydrierung (siehe z.B. Tetrahedron 42 (1986), 5869) oder durch Anlagerung eines Alkohols an die Cyanogruppe im wasserfreien Medium und Ammonolyse der Iminoestergruppierung.

### Beispiele

Die Produkte wurden über Massenspektren und/oder NMR-Spektren identifiziert.

### Beispiel 1:

### (S)-(4-(4-Cyanophenyl)-4 methyl-2,5-dioxoimidazolidin-1-yl)essigsäure

### 1a. (R,S)-4-(4-Bromophenyl)-4-methyl-2,5-dioxoimidazolidin

49.8 g (0.25 Mol) 4-Bromacetophenon, 21.2 g (0.325 Mol) Kaliumcyanid und 211.4 g (2.2 Mol) Ammoniumcarbonat wurden in 1.0 l einer wäßrigen Ethanol-Lösung (0.5 l destilliertes Wasser und 0.5 l Ethanol) suspendiert. Die Suspension wurde bei 60°C gerührt, bis sich dünnschichtchromatographisch kein Ausgangsmaterial mehr nachweisen ließ (8 Stunden). Man ließ auf Raumtemperatur abkühlen. Der pH der Lösung wurde mit halbkonzentrierter Salzsäure auf pH = 6.3 eingestellt. Das Produkt fiel als weißer Niederschlag aus. Der Ansatz wurde über Nacht bei 4°C stehen gelassen. Der weiße Niederschlag wurde abgesaugt, mit Wasser gewaschen und im Vakuum über Phosphorpentoxid getrocknet.
Ausbeute: 65.3 g eines weißen Feststoffes (97%).

FAB-MS: 269 (M + H)⁺

### 1b. (R,S)-2-Amino-2-(4-bromophenyl)propionsäure

5.3 g (20 mMol) (R,S)-4-(4-Bromophenyl)-4-methyl-2,5-dioxoimidazolidin wurden in 50 ml 3N Natronlauge suspendiert. Die Suspension wurde im Autoklaven 1 Stunde bei 145°C bei einem Stickstoffüberdruck von 10 bar erhitzt. Die abgekühlte Reaktionslösung wurde mit 150 ml Wasser verdünnt und unter starkem Rühren mit Essigsäure unter Eiskühlung auf einen pH von 4 gebracht. Es wurde bei 0°C für 2 Stunden gerührt. Der Niederschlag wurde abgesaugt, mit Wasser gewaschen und im Vakuum über Phosphorpentoxid getrocknet.
Ausbeute: 3.65 g eines weißen Feststoffes (75%).

FAB-MS: 244 (M + H)⁺

### 1c. (R,S)-2-Amino-2-(4-bromophenyl)propionsäureethylester

27.3 g (112.3 mMol) (R,S)-2-Amino-2-(4-bromophenyl)propionsäure wurden in 150 ml 9.8 N ethanolischer Chlorwasserstoff-Lösung suspendiert. Man erhitzte 18 Stunden am Rückfluß, setzte nochmals 50 ml 9.8 N ethanolischer Chlorwasserstoff-Lösung zu und erhitzte weitere 5 Stunden am Rückfluß. Die Lösung wurde eingeengt und der Rückstand zwischen Essigsäureethylester und gesättigter Natriumbicarbonat-Lösung verteilt. Die organische Phase wurde mit Wasser gewaschen und über Natriumsulfat getrocknet. Das Rohprodukt (23.22 g) wurde im Hochvakuum zur Reinigung destilliert (Sdp. = 129 - 130°C bei 2 Torr).
Ausbeute: 20.7 g (68 %).

FAB-MS: 272 (M + H)⁺

### 1d. (S)-2-Amino-2-(4-bromophenyl)propionsäureethylester

44.3 g (163 mMol) (R,S)-2-Amino-2-(4-bromophenyl)propionsäureethylester und 24.8 g D-(-)-Mandelsäure (163 mMol) wurden in 138 ml Isopropanol bei Raumtemperatur gelöst. Man setzte 414 ml Diisopropylether zu und kühlte über Nacht bei 0°C. Der ausgefallene Niederschlag wurde abgesaugt. Das erhaltene Salz wurde noch zwei weitere Mal in der gleichen Weise umkristallisiert. Es wurden 20 g enantiomerenreines Salz erhalten ([α]_{D} = - 14° (c = 1; 2.15 N ethanolische Chlorwasserstoff-Lösung; 22°C). Das Salz wurde zwischen Essigsäureethylester und wäßriger Natriumbicarbonat-Lösung verteilt. Die organische Phase wurde mit Wasser gewaschen, über Magnesiumsulfat getrocknet und im Vakuum getrocknet. Die Enantiomereneinheit wurde nach Derivatisierung mit R(-)-α-Methoxy-α-(trifluormethyl)-phenylessigsäurechlorid (Mosher-Reagenz) durch HPLC zu größer 99% ee bestimmt.
Ausbeute: 12.5 g (28 %).
[α]_{D} = + 52.7° (c = 1; 2.15 N ethanolische Chlorwasserstoff-Lösung; 22°C).

FAB-MS: 272 (M + H)⁺

### 1e. N-((S)-1-(4-Bromophenyl)-1-(ethoxycarbonyl)ethyl)-N'-(ethoxycarbonylmethyl)harnstoff

12.4 g (45.6 mMol) (S)-2-Amino-2-(4-bromophenyl)propionsäureethylester wurden in 70 ml Methylenchlorid gelöst. Man tropfte bei 0°C innerhalb von 15 Minuten eine Lösung von 5.11 ml (45.6 mMol) Isocyanatoessigsäureethylester in 35 ml Methylenchlorid zu. Es wurde 2 Stunden bei 0°C gerührt und dann eingeengt.
Ausbeute: 18.1 g (99%).
[α]_{D} = + 10.7° (c = 1; 2.15 N ethanolische Chlorwasserstoff-Lösung; 22°C)

FAB-MS: 401 (M + H)⁺

### 1f. (S)-(4-(4-Bromophenyl)-4-methyl-2,5-dioxoimidazolidin-1-yl)essigsäure

18 g (44.9 mMol) N-((S)-1-(4-Bromophenyl)-1-(ethoxycarbonyl)ethyl)-N'-(ethoxycarbonylmethyl)harnstoffwurden mit 180 ml 6 N Chlorwasserstoff-Lösung versetzt. Das Reaktionsgemisch wurde 10 Stunden unter Rückfluß zum Sieden erhitzt. Man ließ auf 0°C abkühlen und saugte das ausgefallene Reaktionsprodukt ab. Es wurde mit Wasser nachgewaschen und im Vakuum über Phosphorpentoxid getrocknet.
Ausbeute: 11.4 g (78 %).
[α]_{D} = + 32.8° (c = 1; 2.15 N ethanolische Chlorwasserstoff-Lösung; 22°C).

FAB-MS: 327 (M + H)⁺

### 1g. (S)-(4-(4-Cyanophenyl)-4-methyl-2,5-dioxoimidazolidin-1-yl)essigsäure

11.75 g (35.9 mMol) (S)-(4-(4-Bromophenyl)-4-methyl-2,5-dioxoimidazolidin-1-yl)essigsäure wurden in 90 ml Dimethylformamid gelöst. Man setzte 14.15 g (158 mMol) Kupfer-I-cyanid zu und erhitzte 20 Stunden unter Rühren am Rückfluß. Das Reaktionsgemisch wurde abgekühlt und dann in 300 ml Wasser gegossen. Die wäßrige Phase wurde mit konzentrierter Salzsäure sauer gestellt (pH = 1 - 1.5), 30 Minuten gerührt und über eine Seitzschicht abgesaugt. Die wäßrige Phase wurde dreimal mit Essigsäureethylester extrahiert. Die vereinigten organischen Phasen wurden mit Wasser gewaschen, über Magnesiumsulfat getrocknet und im Vakuum eingeengt.
Ausbeute: 9.3 g (95 %).
[α]_{D} = + 33.4° (c = 1; 2.15 N ethanolische Chlorwasserstoff-Lösung; 22°C).

FAB-MS: 274 (M + H)⁺

### Beispiel 2:

### (R)-(4-(4-Cyanophenyl)-4-methyl-2,5-dioxoimidazolidin-1-yl)essigsäure

(R)-(4-(4-Cyanopheny)-4-methyl-2,5-dioxoimidazolidin-1-yl)essigsäure wurde analog Beispiel 1 erhalten. Dazu wurde die Racematspaltung von (R,S)-2-Amino-2-(4-bromophenyl)propionsäureethylester wie in Beispiel 1d, jedoch an Stelle von D-(-)-Mandelsäure mit L-(+)-Mandelsäure durchgeführt. Der erhaltene (R)-2-Amino-2-(4-bromophenyl)propionsäureethylester wurde dann weiter analog Beispiel 1e-g umgesetzt.

FAB-MS: 274 (M + H)⁺

### Beispiel 3:

### (S)-(4-(4-Cyanophenyl)-4-methyl-2,5-dioxoimidazolidin-1-yl)essigsäuremethylester

FAB-MS: 288 (M + H)⁺

### Beispiel 4:

### (R)-(4-(4-Cyanophenyl)-4-methyl-2,5-dioxoimidazolidin-1-yl)essigsäuremethylester

FAB-MS: 288 (M + H)⁺

### Beispiel 5:

### (S)-(4-(4-(Amino-imino-methyl)phenyl)-4-methyl-2,5-dioxoimidazolidin-1-yl)essigsäure-hydrochlorid

### 5a. (S)-(4-(4-(Ethoxy-imino-methyl)phenyl)-4-methyl-2,5-dioxoimidazolidin-1-yl)essigsäureethylester-hydrochlorid

Eine Lösung von 27.3 g (S)-(4-(4-Cyanophenyl)-4-methyl-2,5-dioxoimidazolidin-1-yl)essigsäure (100 mMol) in 400 ml absolutem Ethanol wurde auf 0°C abgekühlt. Trockenes Chlorwasserstoff-Gas wurde in die Lösung eingeleitet, wobei die Temperatur stets unter 10°C gehalten wurde, bis im IR-Spektrum die Nitril-Bande nicht mehr vorlag. Die ethanolische Lösung wurde eingeengt.
Ausbeute: 38.1 g (99 %).

FAB-MS: 348 (M + H)⁺

### 5b. (S)-(4-(4-(Amino-imino-methyl)phenyl)-4-methyl 2,5-dioxoimidazolidin-1-yl)essigsäureethylester-hydrochlorid

38 g (S)-(4-(4-(Ethoxy-imino-methyl)phenyl)-4-methyl-2,5-dioxoimidazolidin-1-yl)essigsäureethylester-hydrochlorid (99 mMol) wurden in 380 ml Isopropanol suspendiert und mit 115 ml einer 2 N Lösung von Ammoniak in Isopropanol versetzt. Das Reaktionsgemisch wurde 2 Stunden bei 50°C gerührt. Der Ansatz wurde abgekühlt und dann mit 2 l Diethylether versetzt. Der Niederschlag wurde abgesaugt und im Hochvakuum getrocknet.
Ausbeute: 24.8 g (71 %).
[α]_{D} = + 33.1° (c = 1; 2.15 N ethanolische Chlorwasserstoff-Lösung; 22°C).

FAB-MS: 319 (M + H)⁺

### 5c. (S)-(4-(4-(Amino-imino-methyl)phenyl)-4-methyl-2,5-dioxoimidazolidin-1-yl)essigsäure-hydrochlorid

24.7 g (S)-(4-(4-(Amino-imino-methyl)phenyl)-4-methyl-2,5-dioxoimidazolidin-1-yl)essigsäureethylester-hydrochlorid (69.7 mMol) wurden in 375 ml konzentrierter Salzsäure gelöst. Die Lösung wurde 6 Stunden zum Sieden erhitzt und dann eingeengt. Der Rückstand wurde in Wasser gelöst und gefriergetrocknet.
Ausbeute: 21.65 g (95 %).
[α]_{D} = + 42.7° (c = 1; 1 N Salzsäure; 22°C).

FAB-MS: 291 (M + H)⁺

### Beispiel 6:

### (R)-(4-(4-(Amino-imino-methyl)phenyl)-4-methyl-2,5-dioxoimidazolidin-1-yl)essigsäure-hydrochlorid

Die Herstellung von (R)-(4-(4-(Amino-imino-methyl)phenyl)-4-methyl-2,5-dioxoimidazolidin-1-yl)essigsäure erfolgte ausgehend von (R)-(4-(4-(Cyanophenyl)-4-methyl-2,5-dioxo-imidazolidin-1-yl)essigsäure analog zu Beispiel 5.
[α]_{D} = + 42.7° (c = 1; 1 N Salzsäure; 22°C).

FAB-MS: 291 (M + H)⁺

### Beispiel 7:

### (S)-(4-(4-(Amino-imino-methyl)phenyl)-4-methyl-2,5-dioxoimidazolidin-1-yl)essigsäuremethylester-hydrochlorid

FAB-MS: 305 (M + H)⁺

### Beispiel 8:

### (R)-(4-(4-(Amino-imino-methyl)phenyl)-4-methyl-2,5-dioxoimidazolidin-1-yl)essigsäuremethylester-hydrochlorid

FAB-MS: 305 (M + H)⁺

### Beispiel 9:

### (S)-(4-(4-(Amino-imino-methyl)phenyl)-4-methyl-2,5-dioxoimidazolidin-1-yl)essigsäurebenzylester-hydrochlorid

FAB-MS: 381 (M + H)⁺

### Beispiel 10:

### (R)-(4-(4-(Amino-imino-methyl)phenyl)-4-methyl-2,5-dioxoimidazolidin-1-yl)essigsäurebenzylester-hydrochlorid

FAB-MS: 381 (M + H)⁺

### Beispiel 11:

### (S)-(4-(4-(Amino-imino-methyl)phenyl)-4-methyl-2,5-dioxoimidazolidin-1-yl)essigsäure-tert.-butylester-hydrochlorid

FAB-MS: 347 (M + H)⁺

### Beispiel 12:

### (R)-(4-(4-(Amino-imino-methyl)phenyl)-4-methyl-2,5-dioxoimidazolidin-1-yl)essigsäure-tert.-butylester-hydrochlorid

FAB-MS: 347 (M + H)⁺

### Beispiel 13:

### (S)-(4-(4-(Benzyloxycarbonylamino-imino-methyl)phenyl)-4-methyl-2,5-dioxoimidazolidin-1-yl)essigsäure

FAB-MS: 425 (M + H)⁺

### Beispiel 14:

### (R)-(4-(4-(Benzyloxycarbonylamino-imino-methyl)phenyl)-4-methyl-2,5-dioxoimidazolidin-1-yl)essigsäure

FAB-MS: 425 (M + H)⁺

### Beispiel 15:

### (S)-4-(4-(Amino-imino-methyl)phenyl)-4-methyl-2,5-dioxoimidazolidin-hydrochlorid

FAB-MS: 233 (M + H)⁺

### Beispiel 16:

### (R)-4-(4-(Amino-imino-methyl)phenyl)-4-methyl-2,5-dioxoimidazolidin-hydrochlorid

FAB-MS: 233 (M + H)⁺

### Beispiel 17:

### (S)-(4-(4-(Aminomethyl)phenyl)-4-methyl-2,5-dioxoimidazolidin-1-yl)essigsäure

FAB-MS: 278 (M + H)⁺

### Beispiel 18:

### (R)-(4-(4-(Aminomethyl)phenyl)-4-methyl-2,5-dioxoimidazolidin-1-yl)essigsäure

FAB-MS: 278 (M + H)⁺

### Beispiel 19:

### (S)-(4-(4-(tert.-Butoxycarbonylaminomethyl)phenyl)-4-methyl-2,5-dioxoimidazolidin-1-yl)essigsäure

FAB-MS: 378 (M + H)⁺

### Beispiel 20:

### (R)-(4-(4-(tert.-Butoxycarbonylaminomethyl)phenyl)-4-methyl-2,5-dioxoimidazolidin-1-yl)essigsäure

FAB-MS: 378 (M + H)⁺

### Beispiel 21:

### (S)-(4-(4-Guanidinophenyl)-4-methyl-2,5-dioxoimidazolidin-1-yl)essigsäure

FAB-MS: 306 (M + H)⁺

### Beispiel 22:

### (R)-(4-(4-Guanidinophenyl)-4-methyl-2,5-dioxoimidazolidin-1-yl)essigsäure

FAB-MS: 306 (M + H)⁺

### Beispiel 23:

### (S)-(4-(4-(Benzyloxycarbonylguanidino)phenyl)-4-methyl-2,5-dioxoimidazolidin-1-yl)essigsäure

FAB-MS: 440 (M + H)⁺

### Beispiel 24:

### (R)-(4-(4-(Benzyloxycarbonylguanidino)phenyl)-4-methyl-2,5-dioxoimidazolidin-1-yl)essigsäure

FAB-MS: 440 (M + H)⁺

### Beispiel 25:

### (S)-(4-(4-(Amino-imino-methyl)phenyl)-4-ethyl-2,5-dioxoimidazolidin-1-yl)essigsäure

FAB-MS: 305 (M + H)⁺

### Beispiel 26:

### (R)-(4-(4-(Amino-imino-methyl)phenyl)-4-ethyl-2,5-dioxoimidazolidin-1-yl)essigsäure

FAB-MS: 305 (M + H)⁺

### Beispiel 27:

### (S)-(4-(4-(Amino-imino-methyl)phenyl)-4-cyclopropyl-2,5-dioxoimidazolidin-1-yl)essigsäure

FAB-MS: 317 (M + H)⁺

### Beispiel 28:

### (R)-(4-(4-(Amino-imino-methyl)phenyl)-4-cyclopropyl-2,5-dioxoimidazolidin-1-yl)essigsäure

FAB-MS: 317 (M + H)⁺

### Beispiel 29:

### (S)-(4-(4-(Amino-imino-methyl)phenyl)-4-tert.-butyl-2,5-dioxoimidazolidin-1-yl)essigsäure

FAB-MS: 333 (M + H)⁺

### Beispiel 30:

### (R)-(4-(4-(Amino-imino-methyl)phenyl)-4-tert.-butyl-2,5-dioxoimidazolidin-1-yl)essigsäure

FAB-MS: 333 (M + H)⁺

### Beispiel 31:

### (S)-(4-(4-(Amino-imino-methyl)phenyl)-4-benzyl-2,5-dioxoimidazolidin-1-yl)essigsäure

FAB-MS: 367 (M + H)⁺

### Beispiel 32:

### (R)-(4-(4-(Amino-imino-methyl)phenyl)-4-benzyl-2,5-dioxoimidazolidin-1-yl)essigsäure

FAB-MS: 367 (M + H)⁺

### Beispiel 33:

### (S)-(4-(4-(Amino-imino-methyl)phenyl)-3,4-dimethyl-2,5-dioxoimidazolidin-1-yl)essigsäure-hydrochlorid

### 33a. (S)-(4-(4-Cyanophenyl)-3,4-dimethyl-2,5-dioxoimidazolidin-1-yl)essigsäuremethylester

3 g (S)-(4-(4-Cyanophenyl)-4-methyl-2,5-dioxoimidazolidin-1-yl)essigsäuremethylester (10.4 mMol) wurden unter Argon in 15 ml wasserfreiem Dimethylformamid gelöst. Im Argon-Gegenstrom wurden 11.4 mMol Natriumhydrid in Form einer Dispersion in Mineralöl zugegeben. Das Reaktionsgemisch wurde 15 Minuten bei Raumtemperatur gerührt. Anschließend versetzte man mit 721 µl Methyliodid (11.4 mMol). Es wurde 4 Stunden bei Raumtemperatur gerührt und dann über Nacht bei Raumtemperatur stehen gelassen. Die Lösung wurde konzentriert. Zur Reinigung wurde die Substanz an Kieselgel mit Methylenchlorid/Essigsäureethylester (9.5:0.5) chromatographiert. Die Fraktionen mit der reinen Substanz wurden eingeengt.
Ausbeute: 2.14 g Öl (68 %).

FAB-MS: 302 (M + H)⁺

### 33b. (S)-(4-(4-(Ethoxy-imino-methyl)phenyl)-3,4-dimethyl-2,5-dioxoimidazolidin-1-yl)essigsäuremethylesterhydrochlorid

Eine Lösung von 2.56 g (S)-(4-(4-Cyanophenyl)-3,4-dimethyl-2,5-dioxoimidazolidin-1-yl)essigsäuremethylester (8.5 mMol) in 40 ml absolutem Ethanol wurde auf 0°C abgekühlt. Trockenes Chlorwasserstoff-Gas wurde in die Lösung eingeleitet, wobei die Temperatur stets unter 10°C gehalten wurde, bis im IR-Spektrum die Nitril-Bande nicht mehr vorlag. Die ethanolische Lösung wurde auf 20 ml eingeengt und mit 200 ml Diethylether versetzt. Die Suspension wurde eingeengt und im Hochvakuum getrocknet.
Ausbeute: 2.27 g (76 %).

FAB-MS: 348 (M + H)⁺

### 33c. (S)-(4-(4-(Amino-imino-methyl)phenyl)-3,4-dimethyl-2,5-dioxoimidazolidin-1-yl)essigsäuremethylesterhydrochlorid

2,26 g (S)-(4-(4-(Ethoxy-imino-methyl)phenyl)-3,4-dimethyl-2,5-dioxoimidazolidin-1-yl)essigsäuremethylesterhydrochlorid (6.4 mMol) wurden in 25 ml Isopropanol suspendiert und mit 7.2 ml einer 2 N Lösung von Ammoniak in Isopropanol versetzt. Das Reaktionsgemisch wurde 2.5 Stunden bei 50°C gerührt. Der Ansatz wurde abgekühlt und dann mit 200 ml Diethylether versetzt. Der Niederschlag wurde abgesaugt und im Hochvakuum getrocknet.
Ausbeute: 1.03 g (45 %).

FAB-MS: 319 (M + H)⁺

### 33d. (S)-(4-(4-(Amino-imino-methyl)phenyl)-3,4-dimethyl-2,5-dioxoimidazolidin-1-yl)essigsäure-hydrochlorid

1 g (S)-(4-(4-(Amino-imino-methyl)phenyl)-3,4-dimethyl-2,5-dioxoimidazolidin-1-yl)essigsäuremethylesterhydrochlorid (2.8 mMol) wurden in 20 ml konzentrierter Salzsäure gelöst. Die Lösung wurde 6 Stunden zum Sieden erhitzt und dann eingeengt.
Ausbeute: 770 mg (81 %).

FAB-MS: 305 (M + H)⁺

### Beispiel 34:

### (R)-(4-(4-(Amino-imino-methyl)phenyl)-3,4-dimethyl-2,5-dioxoimidazolidin-1-yl)essigsäure-hydrochlorid

Die Herstellung von (R)-(4-(4-(Amino-imino-methyl)-phenyl)-3,4-dimethyl-2,5-dioxoimidazolidin-1-yl)essigsäure-hydrochlorid erfolgt ausgehend von (R)-(4-(4-Cyanophenyl)-4-methyl-2,5-dioxoimidazolidin-1-yl)essigsäure analog zu Beispiel 33.

FAB-MS: 305 (M + H)⁺

### Beispiel 35:

### (S)-(4-(4-(Amino-imino-methyl)phenyl)-3-ethyl-4-methyl-2,5-dioxoimidazolidin-1-yl)essigsäure

FAB-MS: 319 (M + H)⁺

### Beispiel 36:

### (R)-(4-(4-(Amino-imino-methyl)phenyl)-3-ethyl-4-methyl-2,5-dioxoimidazolidin-1-yl)essigsäure

FAB-MS: 319 (M + H)⁺

### Beispiel 37:

### (S)-(4-(4-(Amino-imino-methyl)phenyl)-3-benzyl-4-methyl-2,5-dioxoimidazolidin-1-yl)essigsäure

FAB-MS: 381 (M + H)⁺

### Beispiel 38:

### (R)-(4-(4-(Amino-imino-methyl)phenyl)-3-benzyl-4-methyl-2,5-dioxoimidazolidin-1-yl)essigsäure

FAB-MS: 381 (M + H)⁺

### Beispiel 39:

### (S)-(4-(4-(Amino-imino-methyl)phenyl)-3-pentafluorbenzyl-4-methyl-2,5-dioxoimidazolidin-1-yl)essigsäure

FAB-MS: 471 (M + H)⁺

### Beispiel 40:

### (R)-(4-(4-(Amino-imino-methyl)phenyl)-3-pentafluorbenzyl-4-methyl-2,5-dioxoimidazolidin-1-yl)essigsäure

FAB-MS: 471 (M + H)⁺

### Beispiel 41:

### (S)-(4-(4-(Amino-imino-methyl)phenyl)-3-(4-tert.-butyl)-4-methyl-2,5-dioxoimidazolidin-1-yl)essigsäure

FAB-MS: 437 (M + H)⁺

### Beispiel 42:

### (R)-(4-(4-(Amino-imino-methyl)phenyl)-3-(4-tert.-butylbenzyl)-4-methyl-2,5-dioxoimidazolidin-1-yl)essigsäure

FAB-MS: 437 (M + H)⁺

### Beispiel 43:

### (S)-(4-(4-(Amino-imino-methyl)phenyl)-3-(4-nitrobenzyl)-4-methyl-2,5-dioxoimidazolidin-1-yl)essigsäure

FAB-MS: 426 (M + H)⁺

### Beispiel 44:

### (R)-(4-(4-(Amino-imino methyl)phenyl)-3-(4-nitrobenzyl)-4-methyl-2,5-dioxoimidazolidin-1-yl)essigsäure

FAB-MS: 426 (M + H)⁺

### Beispiel 45:

### (S)-(4-(4-(Amino-imino-methyl)phenyl)-3-(3,5-dimethylbenzyl)-4-methyl-2,5-dioxoimidazolidin-1-yl)essigsäure

FAB-MS: 409 (M + H)⁺

### Beispiel 46:

### (R)-(4-(4-(Amino-imino-methyl)phenyl)-3-(3,5-dimethylbenzyl)-4-methyl-2,5-dioxoimidazolidin-1-yl)essigsäure

FAB-MS: 409 (M + H)⁺

### Beispiel 47:

### (S)-(4-(4-(Amino-imino-methyl)phenyl)-3-(2-naphthylmethyl)-4-methyl-2,5-dioxoimidazolidin-1-yl)essigsäure

FAB-MS: 431 (M + H)⁺

### Beispiel 48:

### (R)-(4-(4-(Amino-imino-methyl)phenyl)-3-(2-naphthylmethyl)-4-methyl-2,5-dioxoimidazolidin-1-yl)essigsäure

FAB-MS: 431 (M + H)⁺

### Beispiel 49:

### (R oder S)-4-(4-(Amino-imino-methyl)phenyl)-4-methyl-2,5-dioxoimidazolidin-hydrochlorid (Enantiomer I)

### 49a. (R,S)-2-Amino-2-(4-cyanophenyl)propionitril

In einem 1 Liter-Rundkolben wurden 20 g (400 mMol) Natriumcyanid in 40 ml Wasser, 23.56 g (440 mMol) Ammoniumchlorid in 56 ml 35°C warmem Wasser und 53.6 ml konz. Ammoniak vorgelegt. Man gab unter Rühren 58.08 g (400 mMol) 4-Cyanoacetophenon in 120 ml 95 %igem Ethanol zu, erhitzte 5 Stunden auf 60°C und ließ das Gemisch über Nacht bei Raumtemperatur stehen. Der Niederschlag wurde abgesaugt, mit Wasser gewaschen und über Phosphorpentoxid im Hochvakuum getrocknet.
Ausbeute: 46.1 g blaßgelber Feststoff.
Aus der Mutterlauge wurden weitere 5.1 g Produkt erhalten.
Gesamtausbeute: 51.2 g (75%).

FAB-MS: 172 (M + H)⁺

Die Substanz wurde ohne weitere Reinigung direkt wie unter 49b. beschrieben umgesetzt.

### 49b. (R,S)-2-Amino-2-(4-cyanophenyl)propionsäureamid

51.2 g (300 mMol) (R,S)-2-Amino-2-(4-cyanophenyl)propionitril wurden in 400 ml konz. Salzsäure 2.5 Stunden bei 30°C gerührt. Anschließend stellte man unter Eiskühlung mit konz. Natronlauge pH 1 ein, saugte den Niederschlag ab und extrahierte die wäßrige Phase dreimal mit jeweils 300 ml Essigsäureethylester. Die Wasserphase wurde mit konz. Natronlauge auf pH 12 gestellt und dreimal mit jeweils 400 ml Dichlormethan extrahiert. Die vereinigten Dichlormethanphasen wurden über Natriumsulfat getrocknet und eingeengt. Der Rückstand wurde mit Methyl-tert.-butylether/Methanol (9:1) an Kieselgel chromatographiert.
Ausbeute: 21.2 g (37 %) farbloser Feststoff.
Schmelzpunkt 120°C.

FAB-MS: 190 (M + H)⁺

### 49c. (R oder S)-2-Amino-2-(4-cyanophenyl)propionsäureamid

Man löste 10 g (52.8 mMol) (R,S)-2-Amino-2-(4-cyanophenyl)propionsäureamid in 158 ml Methanol, gab 7.92 g (52.8 mMol) L-Weinsäure hinzu, erhitzte bis eine klare Lösung entstanden war und ließ über Nacht bei 0°C kristallisieren. Der Niederschlag wurde abgesaugt, mit wenig eiskaltem Methanol und anschließend Diethylether gewaschen und aus Methanol umkristallisiert.
Ausbeute: 5.2 g (29 %) farbloser Feststoff.

Zur Freisetzung des (R oder S)-2-Amino-2-(4-cyanophenyl)-propionsäureamids aus dem Weinsäuresalz wurde das Salz in Wasser gelöst, mit Natriumhydrogencarbonat neutralisiert und die freie Base dreimal mit jeweils 50 ml Methylenchlorid extrahiert.
Ausbeute: 2.75 g (95 %) an (R oder S)-2-Amino-2-(4-cyanophenyl)propionsäureamid, farbloser Feststoff,
[α]_{D} = - 13.8° (c = 0.5; H₂O; 22°C).

FAB-MS: 190 (M + H)⁺

Die Enantiomerenreinheit wurde nach Derivatisierung mit R(-)-α-Methoxy-α-(trifluormethyl)phenylessigsäurechlorid (Mosher-Reagenz) an LiChrosorb® vorhanden Si60 (Merck) mit Hexan/Dimethoxyethan/Ethanol (74:20:8) zu 96 % ee bestimmt.

### 49d. (R oder S)-4-(4-Cyanophenyl)-4-methyl-2,5-dioxoimidazolidin

(R oder S)-4-(4-Cyanophenyl)-4-methyl-2,5-dioxoimidazolidin wurde aus (R oder S)-2-Amino-2-(4-cyanophenyl)propionsäureamid mit Dimethylcarbonat analog der in der EP-A-173522 angegebenen Vorschrift hergestellt.

FAB-MS: 216 (M + H)⁺

### 49e. (R oder S)-4-(4-(Amino-imino-methyl)phenyl)-4-methyl-2,5-dioxoimidazolidin-hydrochlorid

Die Herstellung von (R oder S)-4-(4-(Amino-imino-methyl)phenyl)-4-methyl-2,5-dioxoimidazolidin-hydrochlorid erfolgte aus (R oder S)-4-(4-Cyanophenyl)-4-methyl-2,5-dioxoimidazolidin analog Beispiel 5.

FAB-MS: 233 (M + H)⁺

### Beispiel 50:

### (S oder R)-4-(4-(Amino-imino-methyl)phenyl)-4-methyl-2,5-dioxoimidazolidin-hydrochlorid (Enantiomer II; die Verbindungen der Beispiele 49 und 50 sind enantiomer zueinander)

### 50a. (S oder R)-2-Amino-2-(4-cyanophenyl)propionsäureamid

(S oder R)-2-Amino-2-(4-cyanophenyl)propionsäureamid wurde durch Racematspaltung von (R,S)-2-Amino-2-(4-cyanophenyl)propionsäureamid mit D-Weinsäure analog Beispiel 49c gewonnen.
Aus 3.7 g (19.6 mMol)(R,S)-2-Amino-2-(4-cyanophenyl)propionsäureamid (Herstellung siehe Beispiel 49a, b) erhielt man nach Freisetzen der Base aus dem Salz mit D-Weinsäure 1.05 g (28 %) (S oder R)-2-Amino-2-(4-cyanophenyl)propionsäureamid;
[α]_{D} = + 13.9° (c = 0.5; H₂O; 22°C).

FAB-MS: 190 (M + H)⁺

### 50b. (S oder R)-4-(4-(Amino-imino-methyl)phenyl)-4-methyl-2,5-dioxoimidazolidin-hydrochlorid

Die Herstellung von (S oder R)-4-(4-(Amino-imino-methyl)phenyl)-4-methyl-2,5-dioxoimidazolidin-hydrochlorid erfolgte aus (S oder R)-2-Amino-2-(4-cyanophenyl)propionsäureamid wie in Beispiel 49d, e erwähnt.

FAB-MS: 190 (M + H)⁺

### Beispiel 51: (Umsetzung zum pharmazeutischen Wirkstoff)

### (S)-3-(((S)-4-(4-(Amino-imino-methyl)phenyl)-4-methyl-2,5-dioxoimidazolidin-1-yl)acetylamino)-3-phenylpropionsäureethylester-hydrochlorid

Zu einer Lösung von 653 mg (S)-(4-(4-(Amino-imino-methyl)phenyl)-4-methyl-2,5-dioxoimidazolidin-1-yl)essigsäure-hydrochlorid (2 mMol) (siehe Beispiel 5), 386 mg (S)-3-Amino-3-phenylpropionsäureethylester (2 mMol) und 270 mg Hydroxybenztriazol in 10 ml Dimethylformamid wurden 440 mg Dicyclohexylcarbodiimid (2 mMol) gegeben. Man ließ eine Stunde bei 0°C und 3 Stunden bei Raumtemperatur rühren und anschließend den Ansatz über Nacht stehen. Der Niederschlag wurde abgesaugt und das Filtrat eingeengt. Zur Reinigung des Produkts wurde der Rückstand (1.8 g) an Sephadex LH 20 mit einer Mischung aus Eisessig, n-Butanol und Wasser chromatographiert. Die Fraktionen mit der reinen Substanz wurden eingeengt. Der Rückstand wurde in verdünnter Salzsäure gelöst und gefriergetrocknet.
Ausbeute: 597 mg (59.5 %)
[α]_{D} = - 57° (c = 1; H₂O; 29°C).

### Beispiel 52: (Umsetzung zum pharmazeutischen Wirkstoff)

### (S)-3-(((S)-4-(4-(Amino-imino-methyl)phenyl)-4-methyl-2,5-dioxoimidazolidin-1-yl)acetylamino)-3-phenylpropionsäureethylester-hydrochlorid

### 52a. (S)-3-(((S)-4-(4-Cyanophenyl)-4-methyl-2,5-dioxoimidazolidin-1-yl)acetylamino)-3-phenylpropionsäureethylester

Zu einer Lösung von 546 mg (S)-(4-(4-Cyanophenyl)-4-methyl-2,5-dioxoimidazolidin-1-yl)essigsäure (2 mMol) (siehe Beispiel 1) und 459 mg (S)-3-Amino-3-phenylpropionsäureethylester-hydrochlorid (2 mmol) in 10 ml Ethylacetat wurden bei 20°C 462 mg N-Ethylmorpholin (4 mmol) gegeben. Man ließ 15 min bei 20°C rühren, gab dann bei dieser Temperatur 656 mg TOTU (O-((Cyan-ethoxycarbonyl-methylen)amino)-N,N,N',N'-tetramethyluroniumtetrafluoroborat) (2 mmol) zu und ließ 2 Stunden bei 20°C nachrühren. Nach Zugabe von 10 ml Wasser wurde weitere 30 Minuten nachgerührt, nach anschließender Zugabe von 10 ml Methyl-tert-butylether wurde nochmals 2 Stunden bei 10°C gerührt und die weiße Suspension abgesaugt. Das Produkt wurde mit einem Gemisch aus Wasser und Ethanol (1:1), dann mit Wasser gewaschen und im Vakuum getrocknet.
Ausbeute: 762 mg (85 %)
FAB-MS: 449 (M + H)⁺
Schmp. 204 - 205°C
[α]_{D} = - 67.8° (c = 1; CH₃OH; 20°C).

### 52b. (S)-3-(((S)-4-(4-(Amino-imino-methyl)phenyl)-4methyl-2,5-dioxoimidazolidin-1-yl)acetylamino)-3-phenylpropionsäureethylester-hydrochlorid

Die Cyanphenylverbindung des Beispiels 52 a wurde entsprechend der Vorschrift in Tetrahedron 42(1986), 5869 über das Amidoxim in die (Amino-imino-methyl)phenylverbindung (Wirkstoff des Beispiels 51) überführt.

### Beispiel 53: (Umsetzung zum pharmazeutischen Wirkstoff)

### (S)-3-(((S)-4-(4-(Amino-imino-methyl)phenyl)-4-methyl-2,5-dioxoimidazolidin-1-yl)acetylamino)-3-phenylpropionsäureethylester-Essigsäuresalz

Zu einer Lösung von 26.14 g (S)-(4-(4-(Amino-imino-methyl)phenyl)-4-methyl-2,5-dioxoimidazolidin-1-yl)essigsäure-hydrochlorid (80 mMol) (siehe Beispiel 5), 18.37 g (S)-3-Amino-3-phenylpropionsäureethylester-hydrochlorid (80 mMol) und 10.8 g Hydroxybenztriazol in 400 ml Dimethylformamid wurden bei 0°C 10.4 ml N-Ethylmorpholin (80 mmol) und 17.6 g Dicyclohexylcarbodiimid (80 mol) gegeben. Man ließ eine Stunde bei 0°C und 3 Stunden bei Raumtemperatur rühren und anschließend den Ansatz über Nacht stehen. Der Niederschlag wurde abgesaugt und das Filtrat eingeengt. Zur Reinigung wurde das Rohprodukt (89 g) an Sephadex LH 20 mit einer Mischung aus Eisessig, n-Butanol und Wasser chromatographiert. Die Fraktionen mit der reinen Substanz wurden eingeengt. Der Rückstand wurde in Wasser gelöst und gefriergetrocknet.
Ausbeute: 35 g (83 %) weißes Pulver
[α)_{D} = - 55.3° (c = 1; H₂O; 22°C)
FAB-MS: 466 (M + H)⁺

## Patentansprüche

1. Hydantoinderivate der allgemeinen Formel I, 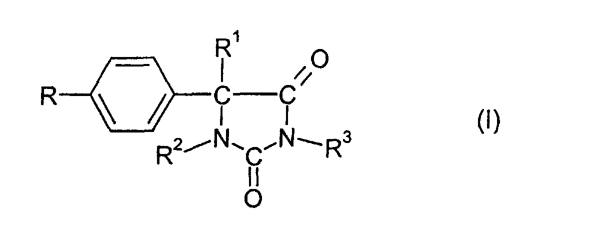 worin
R Cyano, C(=NH)-O-(C₁-C₆)-Alkyl, C(=NH)-NH-X, CH₂-NH-X oder NH-X¹ bedeutet;
X Wasserstoff, (C₁-C₆)-Alkyl, (C₁-C₆)-Alkylcarbonyl, (C₁-C₆)-Alkoxycarbonyl, (C₁-C₁₈)-Alkylcarbonyloxy-(C₁-C₆)-alkoxycarbonyl, gegebenenfalls substituiertes (C₆-C₁₄)-Arylcarbony), gegebenenfalls substituiertes (C₆-C₁₄)-Aryloxycarbonyl, (C₆-C₁₄)-Aryl-(C₁-C₆)-alkoxycarbonyl, das im Arylrest auch substituiert sein kann, (R⁵O)₂P(O), Cyano, Hydroxy, (C₁-C₆)-Alkoxy, (C₆-C₁₄)-Aryl-(C₁-C₆)-alkoxy, das im Arylrest auch substituiert sein kann, oder Amino bedeutet;
X¹ eine der Bedeutungen von X hat oder R'-NH-C(=N-R") bedeutet, wobei R' und R" unabhängig voneinander die Bedeutungen von X haben;
R¹ Methyl, Ethyl, Propyl, Butyl, Pentyl, Hexyl, Isopropyl, Isobutyl, Isopentyl, Neopentyl, isohexyl, 3-Methylpentyl, sec-Butyl, tert-Butyl, tert-Pentyl, im Arylrest gegebenenfalls substituiertes (C₆-C₁₄)-Aryl-(C₁-C₈)-alkyl oder (C₃-C₈)-Cycloalkyl bedeutet;
R² Wasserstoff, (C₁-C₈)-Alkyl, gegebenenfalls substituiertes (C₆-C₁₄)-Aryl, im Arylrest gegebenenfalls substituiertes (C₆-C₁₄)-Aryl-(C₁-C₈)-alkyl oder (C₃-C₈)-Cycloalkyl bedeutet;
R³ Wasserstoff oder CH₂-CO-OR⁴ bedeutet;
R⁴ Wasserstoff, (C₁-C₆)-Alkyl, im Arylrest gegebenenfalls substituiertes (C₆-C₁₄)-Aryl-(C₁-C₆)-alkyl oder gegebenenfalls substituiertes (C₆-C₁₄)-Aryl bedeutet;
R⁵ Wasserstoff, (C₁-C₈)-Alkyl, gegebenenfalls substituiertes (C₆-C₁₄)-Aryl oder (C₆-C₁₄)-Aryl-(C₁-C₈)-alkyl, das im Arylrest auch substituiert sein kann, bedeutet;
wobei aber, wenn R³ für Wasserstoff steht, R nicht für CN, NH₂ oder CH₂-NH₂ stehen kann;
und wobei, wenn die Verbindungen der allgemeinen Formel I hinsichtlich des asymmetrischen Zentrums im Hydantoinring in racemischer Form vorliegen und gleichzeitig R³ für Methoxycarbonylmethyl, R¹ für Methyl und R² für Wasserstoff oder Methyl steht, R nicht für CN oder C(=NH)-OC₂H₅ stehen kann;
und wobei, wenn die Verbindungen der allgemeinen Formel I hinsichtlich des asymmetrischen Zentrums im Hydantoinring in racemischer Form vorliegen und gleichzeitig R³ für Methoxycarbonylmethyl oder Hydroxycarbonylmethyl, R¹ für Methyl und R² für Wasserstoff oder Methyl steht, R nicht für NH₂, CH₂-NH₂, C(=NH)-NH₂, tert-Butoxycarbonylaminomethyl oder Benzyloxycarbonylguanidino stehen kann;
und wobei, wenn die Verbindungen der allgemeinen Formel I hinsichtlich des asymmetrischen Zentrums im Hydantoinring in racemischer Form vorliegen und gleichzeitig R¹ für Methyl und R² und R³ für Wasserstoff steht, R nicht für NH-CO-CH₃ stehen kann;
und wobei eine Arylgruppe, die substituiert ist, einen, zwei oder drei gleiche oder verschiedene Substituenten aus der Reihe (C₁-C₈)-Alkyl, (C₁-C₈)-Alkoxy, Halogen, Nitro, Amino, Trifluormethyl, Hydroxy, Methylendioxy, Cyano, Hydroxycarbonyl, Aminocarbonyl, (C₁-C₄)-Alkoxycarbonyl, Phenyl, Phenoxy, Benzyloxy, (R⁵O)₂P(O), (R⁵O)₂P(O)-O-, Tetrazolyl trägt;
und ihre Salze.

2. Hydantoinderivate gemäß Anspruch 1, worin
R für Cyano oder C(=NH)-NH-X steht;
X für Wasserstoff, (C₁-C₆)-Alkylcarbonyl, (C₁-C₆)-Alkoxycarbonyl oder gegebenenfalls substituiertes (C₆-C₁₄)-Aryl-(C₁-C₆)-alkoxycarbonyl steht;
R¹ für Methyl, Ethyl, Propyl, Butyl, Pentyl, Hexyl, Isopropyl, Isobutyl, Isopentyl, Neopentyl, Isohexyl, 3-Methylpentyl, sec-Butyl, tert-Butyl, tert-Pentyl, im Arylrest gegebenenfalls substituiertes (C₆-C₁₄)-Aryl-(C₁-C₈)-alkyl oder (C₃-C₈)-Cycloalkyl steht;
R² für Wasserstoff, (C₁-C₈)-Alkyl, gegebenenfalls substituiertes Phenyl, im Phenylrest gegebenenfalls substituiertes Phenyl-(C₁-C₈)-alkyl oder (C₃-C₈)-Cycloalkyl steht;
R³ für CH₂-CO-OR⁴ steht;
R⁴ für Wasserstoff, (C₁-C₆)-Alkyl, im Arylrest gegebenenfalls substituiertes (C₆-C₁₄)-Aryl-(C₁-C₆)-aikyl oder gegebenenfalls substituiertes (C₆-C₁₄)-Aryl steht;
wobei aber, wenn die Verbindungen der allgemeinen Formel I hinsichtlich des asymmetrischen Zentrums im Hydantoinring in racemischer Form vorliegen und gleichzeitig R³ für Methoxycarbonylmethyl, R¹ für Methyl und R² für Wasserstoff oder Methyl steht, R nicht für CN stehen kann;
und wobei, wenn die Verbindungen der allgemeinen Formel I hinsichtlich des asymmetrischen Zentrums im Hydantoinring in racemischer Form vorliegen und gleichzeitig R³ für Methoxycarbonylmethyl oder Hydroxycarbonylmethyl, R¹ für Methyl und R² für Wasserstoff oder Methyl steht, R nicht für C(=NH)-NH₂ stehen kann;
und wobei eine Arylgruppe oder Phenylgruppe, die substituiert ist, wie in Anspruch 1 angegeben substituiert ist.

3. Hydantoinderivate gemäß Anspruch 1 und/oder 2, worin
R für Cyano oder C(=NH)-NH₂ steht;
R¹ für (C₁-C₄)-Alkyl, Cyclopropyl oder Benzyl, insbesondere Methyl, steht;
R² für Wasserstoff oder (C₁-C₄)-Alkyl, insbesondere Wasserstoff, steht;
R³ für CH₂-COOH oder CH₂-COO-(C₁-C₄)-Alkyl, insbesondere CH₂-COOH, steht;
wobei aber, wenn die Verbindungen der allgemeinen Formel I hinsichtlich des asymmetrischen Zentrums im Hydantoinring in racemischer Form vorliegen und gleichzeitig R³ für Methoxycarbonylmethyl, R¹ für Methyl und R² für Wasserstoff oder Methyl steht, R nicht für CN stehen kann;
und wobei, wenn die Verbindungen der allgemeinen Formel I hinsichtlich des asymmetrischen Zentrums im Hydantoinring in racemischer Form vorliegen und gleichzeitig R³ für Methoxycarbonylmethyl oder Hydroxycarbonylmethyl, R¹ für Methyl und R² für Wasserstoff oder Methyl steht, R nicht für C(=NH)-NH₂ stehen kann.

4. Hydantoinderivate gemäß einem oder mehreren der Ansprüche 1 bis 3, **dadurch gekennzeichnet, daß** sie hinsichtlich des asymmetrischen Zentrums im Hydantoinring in enantiomerenreiner Form vorliegen, bevorzugt in der S-Konfiguration.

5. Verfahren zur Herstellung von Hydantoinderivaten gemäß einem oder mehreren der Ansprüche 1 bis 4, **dadurch gekennzeichnet, daß** die Aminosäureester der allgemeinen Formel V, 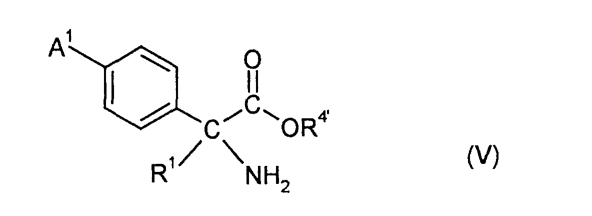 in der A¹ für Halogen, bevorzugt Brom, oder Nitro steht, R¹ die in Anspruch 1 angegebenen Bedeutungen hat und R^{4'} die in Anspruch 1 für R⁴ angegebenen Bedeutungen mit Ausnahme von Wasserstoff hat, mit Isocyanatoessigsäureestern umgesetzt und die erhaltenen Produkte zu den Hydantoinessigsäuren der allgemeinen Formel VIII, 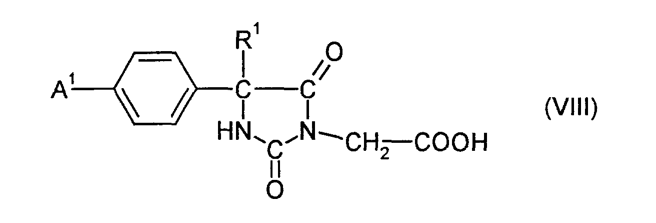 cyclisiert werden, und diese durch Transformation der Gruppe A¹ in den Rest R, der NH-Gruppe in die N-R²-Gruppe und der COOH-Gruppe in die COOR⁴-Gruppe in die Verbindungen der allgemeinen Formel I überführt werden, oder daß, wenn Verbindungen der allgemeinen Formel I, in der R³ die Bedeutung Wasserstoff hat, hergestellt werden sollen, die Verbindungen der allgemeinen Formel V in die Hydantoinderivate der allgemeinen Formel III 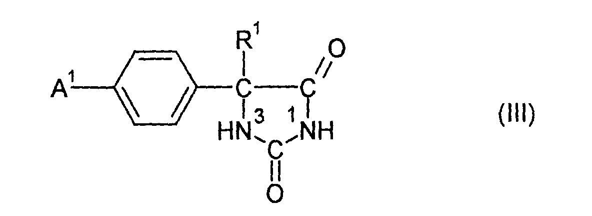 überführt und in diesen die Gruppe A¹ und die N³H-Funktion umgewandelt werden.

6. Verfahren gemäß Anspruch 5, **dadurch gekennzeichnet, daß** Hydantoinderivate hergestellt werden, die hinsichtlich des asymmetrischen Zentrums im Hydantoinring enantiomerenrein sind und bevorzugt die S-Konfiguration aufweisen.

7. Verfahren zur Herstellung von Hydantoinderivaten gemäß einem oder mehreren der Ansprüche 1 bis 4, **dadurch gekennzeichnet, daß** die Aminosäureamide der allgemeinen Formel XI, 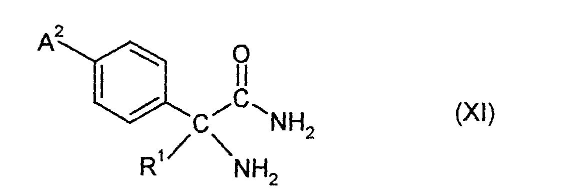 in der A² für Halogen, bevorzugt Brom, Nitro oder Cyano steht und R¹ die in Anspruch 1 angegebenen Bedeutungen hat, mit Isocyanatoessigsäureestern umgesetzt und die erhaltenen Produkte zu den Hydantoinessigsäuren der allgemeinen Formel XIII, 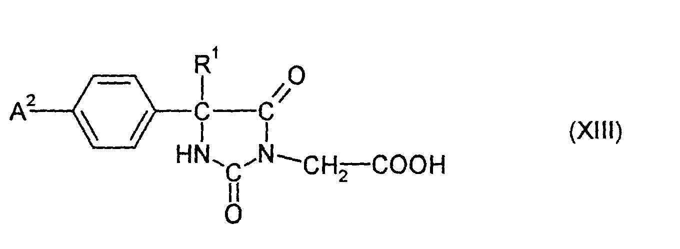 cyclisiert werden, und diese durch Transformation der Gruppe A² in den Rest R, der NH-Gruppe in die N-R²-Gruppe und der COOH-Gruppe in die COOR⁴-Gruppe in die Verbindungen der allgemeinen Formel I überführt werden, oder daß, wenn Verbindungen der allgemeinen Formel I, in der R³ die Bedeutung Wasserstoff hat, hergestellt werden sollen, die Verbindungen der allgemeinen Formel XI in die Hydantoinderivate der allgemeinen Formel XIV 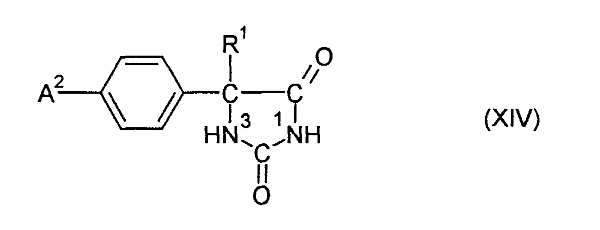 überführt und in diesen die Gruppe A² und die N³H-Funktion umgewandelt werden.

8. Verfahren gemäß Anspruch 7, **dadurch gekennzeichnet, daß** Hydantoinderivate hergestellt werden, die hinsichtlich des asymmetrischen Zentrums im Hydantoinring enantiomerenrein sind und bevorzugt die S-Konfiguration aufweisen.

9. Verwendung von Hydantoinderivaten gemäß einem oder mehreren der Ansprüche 1 bis 4 als Zwischenprodukte für die Herstellung von pharmazeutischen Wirkstoffen.

10. Verwendung gemäß Anspruch 9, **dadurch gekennzeichnet, daß** Wirkstoffe der allgemeinen Formel XVI hergestellt werden, 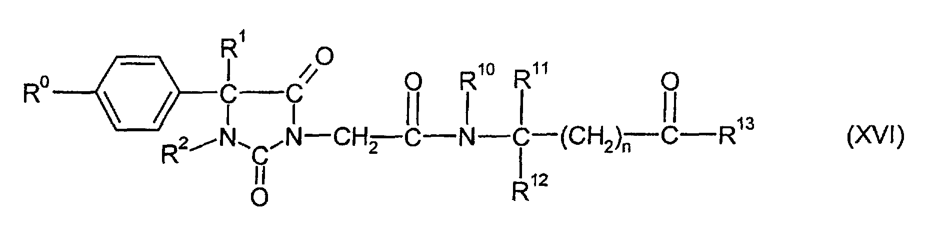 worin
R⁰ C(=NH)-NH-X, CH₂-NH-X oder NH-X¹ bedeutet;
X Wasserstoff, (C₁-C₆)-Alkyl, (C₁-C₆)-Alkylcarbonyl, (C₁-C₆)-Alkoxycarbonyl, (C₁-C₁₈)-Alkylcarbonyloxy-(C₁-C₆)-alkoxycarbonyl, gegebenenfalls substituiertes (C₆-C₁₄)-Arylcarbonyl, gegebenenfalls substituiertes (C₆-C₁₄)-Aryloxycarbonyl, (C₆-C₁₄)-Aryl-(C₁-C₆)-alkoxycarbonyl, das im Arylrest auch substituiert sein kann, (R⁵O)₂P(O), Cyano, Hydroxy, (C₁-C₆)-Alkoxy, (C₆-C₁₄)-Aryl-(C₁-C₆)-alkoxy, das im Arylrest auch substituiert sein kann, oder Amino bedeutet;
X¹ eine der Bedeutungen von X hat oder R'-NH-C(=N-R") bedeutet, wobei R' und R" unabhängig voneinander die Bedeutungen von X haben;
R¹ Methyl, Ethyl, Propyl, Butyl, Pentyl, Hexyl, Isopropyl, Isobutyl, Isopentyl, Neopentyl, Isohexyl, 3-Methylpentyl, sec-Butyl, tert-Butyl, tert-Pentyl, im Arylrest gegebenenfalls substituiertes (C₆-C₁₄)-Aryl-(C₁-C₈)-alkyl oder (C₃-C₈)-Cycloalkyl bedeutet;
R², R¹⁰ und R¹¹ unabhängig voneinander Wasserstoff, (C₁-C₈)-Alkyl, gegebenenfalls substituiertes (C₆-C₁₄)-Aryl, im Arylrest gegebenenfalls substituiertes (C₆-C₁₄)-Aryl-(C₁-C₈)-alkyl oder (C₃-C₈)-Cycloalkyl bedeuten;
R⁵ Wasserstoff, (C₁-C₈)-Alkyl, gegebenenfalls substituiertes (C₆-C₁₄)-Aryl oder (C₆-C₁₄)-Aryl-(C₁-C₈)-alkyl, das im Arylrest auch substituiert sein kann, bedeutet;
R¹² Wasserstoff, (C₁-C₈)-Alkyl, gegebenenfalls substituiertes (C₆-C₁₄)-Aryl, im Arylrest gegebenenfalls substituiertes (C₆-C₁₄)-Aryl-(C₁-C₈)-alkyl, (C₃-C₈)-Cycloalkyl oder 2-, 3- oder 4-Pyridyl bedeutet;
R¹³ Hydroxy, (C₁-C₁₈)-Alkoxy, (C₆-C₁₄)-Aryl-(C₁-C₈)-alkoxy, das im Arylrest auch substituiert sein kann, gegebenenfalls substituiertes (C₆-C₁₄)-Aryloxy, Amino oder Mono- oder Di-((C₁-C₁₈)-alkyl)-amino bedeutet;
n eine ganze Zahl von 0 bis 6 bedeutet;
wobei eine Arylgruppe, die substituiert ist, einen, zwei oder drei gleiche oder verschiedene Substituenten aus der Reihe (C₁-C₈)-Alkyl, (C₁-C₈)-Alkoxy, Halogen, Nitro, Amino, Trifluormethyl, Hydroxy, Methylendioxy, Cyano, Hydroxycarbonyl, Aminocarbonyl, (C₁-C₄)-Alkoxycarbonyl, Phenyl, Phenoxy, Benzyloxy, (R⁵O)₂P(O), (R⁵O)₂P(O)-O-, Tetrazolyl trägt.

11. Hydantoinderivate der allgemeinen Formel XVIII, 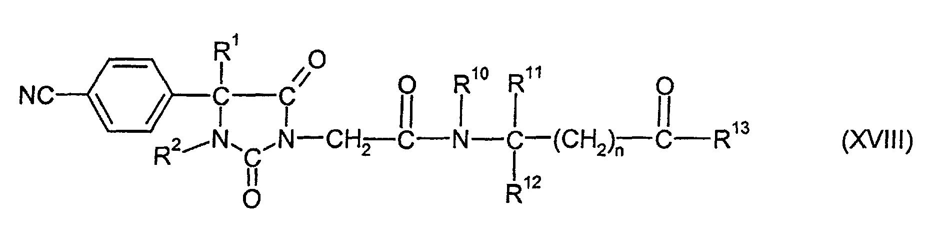 worin
R¹ Methyl, Ethyl, Propyl, Butyl, Pentyl, Hexyl, Isopropyl, Isobutyl, Isopentyl, Neopentyl, Isohexyl, 3-Methylpentyl, sec-Butyl, tert-Butyl, tert-Pentyl, im Arylrest gegebenenfalls substituiertes (C₆-C₁₄)-Aryl-(C₁-C₈)-alkyl oder (C₃-C₈)-Cycloalkyl bedeutet;
R², R¹⁰ und R¹¹ unabhängig voneinander Wasserstoff, (C₁-C₈)-Alkyl, gegebenenfalls substituiertes (C₆-C₁₄)-Aryl, im Aryl rest gegebenenfalls substituiertes (C₆-C₁₄)-Aryl-(C₁-C₈)-alkyl oder (C₃-C₈)-Cycloalkyl bedeuten;
R¹² Wasserstoff, (C₁-C₈)-Alkyl, gegebenenfalls substituiertes (C₆-C₁₄)-Aryl, im Aryl rest gegebenenfalls substituiertes (C₆-C₁₄)-Aryl-(C₁-C₈)-alkyl, (C₃-C₈)-Cycloalkyl oder 2-, 3- oder 4-Pyridyl bedeutet;
R¹³ Hydroxy, (C₁-C₁₈)-Alkoxy, (C₆-C₁₄)-Aryl-(C₁-C₈)-alkoxy, das im Arylrest auch substituiert sein kann, gegebenenfalls substituiertes (C₆-C₁₄)-Aryloxy, Amino oder Mono- oder Di-((C₁-C₁₈)-alkyl)-amino bedeutet;
n eine ganze Zahl von 0 bis 6 bedeutet;
wobei eine Arylgruppe, die substituiert ist, einen, zwei oder drei gleiche oder verschiedene Substituenten aus der Reihe (C₁-C₈)-Alkyl, (C₁-C₈)-Alkoxy, Halogen, Nitro, Amino, Trifluormethyl, Hydroxy, Methylendioxy, Cyano, Hydroxycarbonyl, Aminocarbonyl, (C₁-C₄)-Alkoxycarbonyl, Phenyl, Phenoxy, Benzyloxy, (R⁵O)₂P(O), (R⁵O)₂P(O)-O-, Tetrazolyl trägt;
und ihre Salze.

12. Hydantoinderivate gemäß Anspruch 11, worin,
R¹ für (C₁-C₄)-Alkyl, Cyclopropyl oder Benzyl, bevorzugt für Methyl, steht;
R² für Wasserstoff oder (C₁-C₄)-Alkyl, bevorzugt für Wasserstoff, steht
R¹⁰ für Wasserstoff, (C₁-C₆)-Alkyl oder Benzyl, bevorzugt für Wasserstoff, steht;
R¹¹ für Wasserstoff oder (C₁-C₈)-Alkyl, bevorzugt für Wasserstoff, steht;
R¹² für gegebenenfalls substituiertes (C₆-C₁₄)-Aryl oder Pyridyl, bevorzugt für gegebenenfalls substituiertes Phenyl, besonders bevorzugt für unsubstituiertes Phenyl, steht;
R¹³ für Hydroxy oder (C₁-C₈)-Alkoxy, bevorzugt für (C₁-C₄)-Alkoxy, steht;
n eine ganze Zahl von 0 bis 3, bevorzugt für die Zahlen 1 oder 2, besonders bevorzugt für die Zahl 1, steht;
und worin bevorzugt am asymmetrischen Zentrum im Hydantoinring als auch an dem die Reste R¹¹ und R¹² tragenden Kohlenstoffatom eine einheitliche Konfiguration vorliegt, insbesondere an beiden die S-Konfiguration
wobei eine Arylgruppe oder Phenylgruppe, die substituiert ist, einen, zwei oder drei gleiche oder verschiedene Substituenten aus der Reihe (C₁-C₈)-Alkyl, (C₁-C₈)-Alkoxy, Halogen, Nitro, Amino, Trifluormethyl, Hydroxy, Methylendioxy, Cyano, Hydroxycarbonyl, Aminocarbonyl, (C₁-C₄)-Alkoxycarbonyl, Phenyl, Phenoxy, Benzyloxy, (R⁵O)₂P(O), (R⁵O)₂P(O)-O-, Tetrazolyl trägt.

13. Verfahren zur Herstellung von Hydantoinderivaten gemäß Anspruch 11 und/oder 12, **dadurch gekennzeichnet, daß** Verbindungen der allgemeinen Formel Ib, 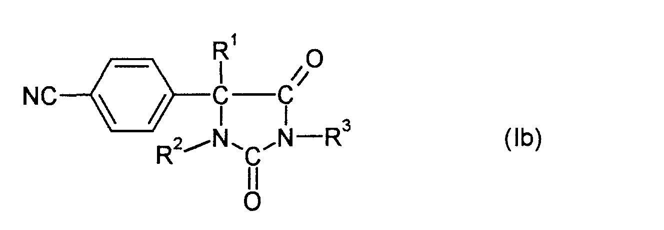 in der R¹ und R² die in Anspruch 11 angegebenen Bedeutungen haben und R³ die in Anspruch 1 angegebenen Bedeutungen mit Ausnahme der Bedeutung Wasserstoff hat, mit Verbindungen der allgemeinen Formel XVII, 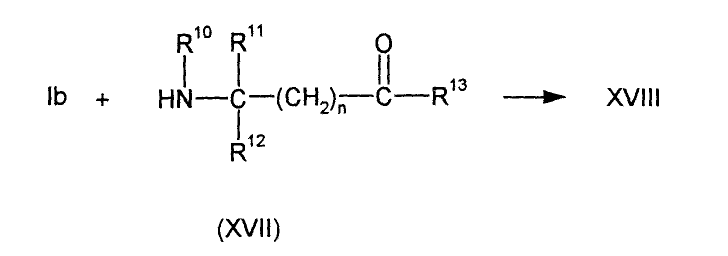 in der R¹⁰ bis R¹³ und n die in Anspruch 11 angegebenen Bedeutungen haben, umgesetzt werden.

14. Verwendung von Hydantoinderivaten gemäß Anspruch 11 und/oder 12 als Zwischenprodukte für die Herstellung von pharmazeutischen Wirkstoffen.

## Claims

1. A hydantoin derivative of the formula I 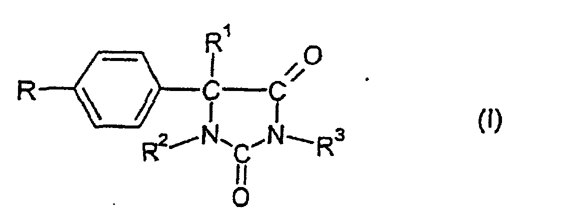 in which
R is cyano, C(=NH)-O-(C₁-C₆)-alkyl, C(=NH)-NH-X, CH₂-NH-X or NH-X¹;
X is hydrogen, (C₁-C₆)-alkyl, (C₁-C₆)-alkylcarbonyl, (C₁-C₆)-alkoxycarbonyl, (C₁-C₁₈)-alkylcarbonyloxy-(C₁-C₆)-alkoxycarbonyl, optionally substituted (C₆-C₁₄)-arylcarbonyl, optionally substituted (C₆-C₁₄)-aryloxycarbonyl, (C₆-C₁₄)-aryl-(C₁-C₆)-alkoxycarbonyl which can also be substituted in the aryl radical, (R⁵O)₂P(O), cyano, hydroxyl, (C₁-C₆)-alkoxy, (C₆-C₁₄)-aryl-(C₁-C₆)-alkoxy which can also be substituted in the aryl radical, or amino;
X¹ has one of the meanings of X or is R'-NH-C(=N-R"), where R' and R" independently of one another have the meanings of X;
R¹ is methyl, ethyl, propyl, butyl, pentyl, hexyl, isopropyl, isobutyl, isopentyl, neopentyl, isohexyl, 3-methylpentyl, sec-butyl, tert-butyl, tent-pentyl, (C₆-C₁₄)-aryl-(C₁-C₈)-alkyl optionally substituted in the aryl radical or (C₃-C₈)-cycloalkyl;
R² is hydrogen, (C₁-C₈)-alkyl, optionally substituted (C₆-C₁₄)-aryl, (C₆-C₁₄)-aryl-(C₁-C₈)-alkyl optionally substituted in the aryl radical or (C₃-C₈)-cycloalkyl;
R³ is hydrogen or CH₂-CO-OR⁴;
R⁴ is hydrogen, (C₁-C₆)-alkyl, (C₆-C₁₄)-aryl-(C₁-C₆)-alkyl optionally substituted in the aryl radical or optionally substituted (C₆-C₁₄)-aryl;
R⁵ is hydrogen, (C₁-C₈)-alkyl, optionally substituted (C₆-C₁₄)-aryl or (C₆-C₁₄)-aryl-(C₁-C₈)-alkyl, which can also be substituted in the aryl radical;
where, however, if R³ is hydrogen, R cannot be CN, NH₂ or CH₂-NH₂;
and where, if the compounds of the formula I are present in racemic form with respect to the asymmetric center in the hydantoin ring and at the same time R³ is methoxycarbonylmethyl, R¹ is methyl and R² is hydrogen or methyl, R cannot be CN or C(=NH)-OC₂H₅;
and where, if the compounds of the formula I are present in racemic form with respect to the asymmetric center in the hydantoin ring and at the same time R³ is methoxycarbonylmethyl or hydroxycarbonylmethyl, R¹ is methyl and R² is hydrogen or methyl, R cannot be NH₂, CH₂-NH₂, C(=NH)-NH₂, tert-butoxycarbonylaminomethyl or benzyloxycarbonylguanidino;
and where, if the compounds of the formula I are present in racemic form with respect to the asymmetric center in the hydantoin ring and at the same time R¹ is methyl and R² and R³ are hydrogen, R cannot be NH-CO-CH₃;
and where an aryl group, which is substituted, carries one, two or three identical or different substituents selected from the group consisting of (C₁-C₈)-alkyl, (C₁-C₈)-alkoxy, halogen, nitro, amino, trifluoromethyl, hydroxyl, methylenedioxy, cyano, hydroxycarbonyl, aminocarbonyl, (C₁-C₄)-alkoxycarbonyl, phenyl, phenoxy, benzyloxy, (R⁵O)₂P(O), (R⁵O)₂P(O)-O- and tetrazolyl;
or its salts.

2. A hydantoin derivative of the formula I as claimed in claim 1, in which
R is cyano or C(=NH)-NH-X;
X is hydrogen, (C₁-C₆)-alkylcarbonyl, (C₁-C₆)-alkoxycarbonyl or optionally substituted (C₆-C₁₄)-aryl-(C₁-C₆)-alkoxycarbonyl;
R¹ is methyl, ethyl, propyl, butyl, pentyl, hexyl, isopropyl, isobutyl, isopentyl, neopentyl, isohexyl, 3-methylpentyl, sec-butyl, tent-butyl, tert-pentyl, (C₆-C₁₄)-aryl-(C₁-C₈)-alkyl optionally substituted in the aryl radical or (C₃-C₈)-cycloalkyl;
R² is hydrogen, (C₁-C₈)-alkyl, optionally substituted phenyl, phenyl-(C₁-C₈)-alkyl optionally substituted in the phenyl radical or (C₃-C₈)-cycloalkyl;
R³ is CH₂-CO-OR⁴;
R⁴ is hydrogen, (C₁-C₆)-alkyl, (C₆-C₁₄)-aryl-(C₁-C₆)-alkyl optionally substituted in the aryl radical or optionally substituted (C₆-C₁₄)-aryl;
where, however, if the compounds of the formula I are present in racemic form with respect to the asymmetric center in the hydantoin ring and at the same time R³ is methoxycarbonylmethyl, R¹ is methyl and R² is hydrogen or methyl, R cannot be CN;
and where, if the compounds of the formula I are present in racemic form with respect to the asymmetric center in the hydantoin ring and at the same time R³ is methoxycarbonylmethyl or hydroxycarbonylmethyl, R¹ is methyl and R² is hydrogen or methyl, R cannot be C(=NH)-NH₂;
and where an aryl group or phenyl group, which is substituted, is substituted as indicated in claim 1.

3. A hydantoin derivative as claimed in claim 1 and/or 2, in which
R is cyano or C(=NH)-NH₂;
R¹ is (C₁-C₄)-alkyl, cyclopropyl or benzyl, in particular methyl;
R² is hydrogen or (C₁-C₄)-alkyl, in particular hydrogen;
R³ is CH₂-COOH or CH₂-COO-(C₁-C₄)-alkyl, in particular CH₂-COOH;
where, however, if the compounds of the formula I are present in racemic form with respect to the asymmetric center in the hydantoin ring and at the same time R³ is methoxycarbonylmethyl, R¹ is methyl and R² is hydrogen or methyl, R cannot be CN;
and where, if the compounds of the formula I are present in racemic form with respect to the asymmetric center in the hydantoin ring and at the same time R³ is methoxycarbonylmethyl or hydroxycarbonylmethyl, R¹ is methyl and R² is hydrogen or methyl, R cannot be C(=NH)-NH₂.

4. A hydantoin derivative as claimed in one or more of claims 1 to 3, wherein it is present in enantiomerically pure form, preferably in the S configuration, with respect to the asymmetric center in the hydantoin ring.

5. A process for the preparation of hydantoin derivatives as claimed in one or more of claims 1 to 4, which comprises reacting the amino acid esters of the formula V 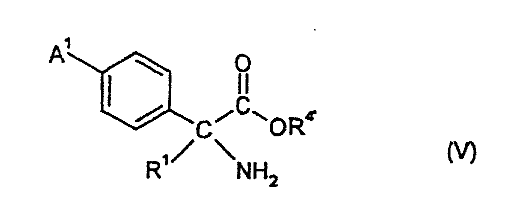 in which A¹ is halogen, preferably bromine, or nitro, R¹ has the meanings indicated in claim 1 and R^{4'} has the meanings indicated in claim 1 for R⁴ with the exception of hydrogen, with isocyanatoacetic acid esters and cyclizing the products obtained to give the hydantoinacetic acids of the formula VIII 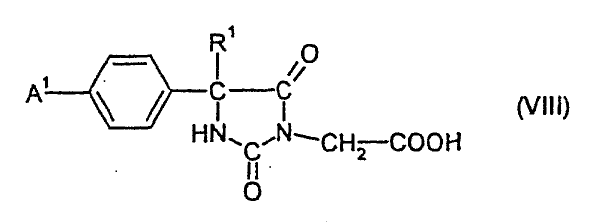 and converting these by a transformation of the group A¹ into the radical R, the NH group into the N-R² group and the COOH group into the COOR⁴ group into the compounds of the formula I, or, if compounds of the formula I in which R³ has the meaning hydrogen are to be prepared, converting the compounds of the formula V into the hydantoin derivatives of the formula III 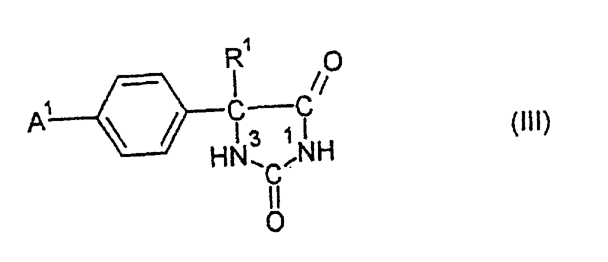 and in these converting the group A¹ and the N³H function.

6. The process as claimed in claim 5, wherein hydantoin derivatives are prepared which are enantiomerically pure with respect to the asymmetric center in the hydantoin ring and preferably have the S configuration.

7. A process for the preparation of hydantoin derivatives as claimed in one or more of claims 1 to 4, which comprises reacting the amino acid amides of the formula XI 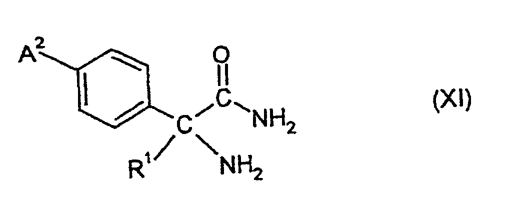 in which A² is halogen, preferably bromine, nitro or cyano and R¹ has the meanings indicated in claim 1, with isocyanatoacetic acid esters and cyclizing the products obtained to give the hydantoinacetic acids of the formula XIII 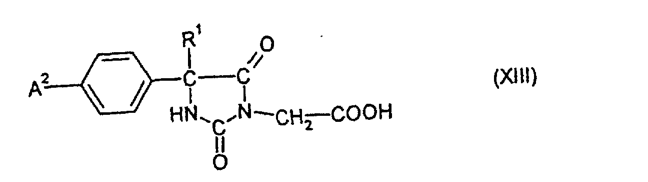 and converting these by transformation of the group A² into the radical R, the NH group into the N-R² group and the COOH group into the COOR⁴ group into the compounds of the formula I, or, if compounds of the formula I in which R³ has the meaning hydrogen are to be prepared, converting the compounds of the formula XI into the hydantoin derivatives of the formula XIV 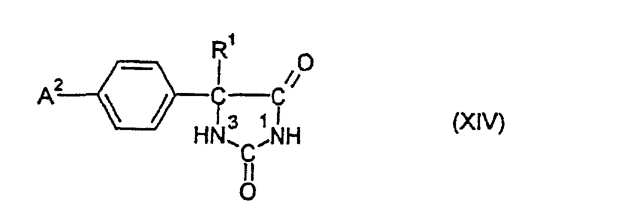 and in these converting the group A² and the N³H function.

8. The process as claimed in claim 7, wherein hydantoin derivatives are prepared which are enantiomerically pure with respect to the asymmetric center in the hydantoin ring and preferably have the S configuration.

9. The use of hydantoin derivatives as claimed in one or more of claims 1 to 4 as intermediates for the preparation of pharmaceutical active compounds.

10. The use as claimed in claim 9, wherein active compounds of the formula XVI are prepared 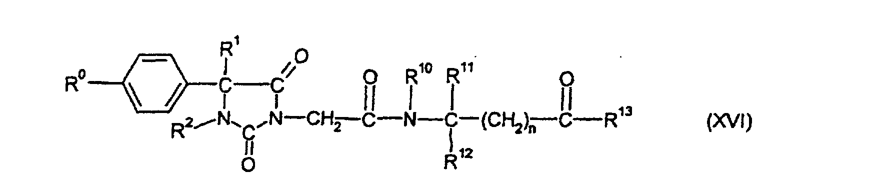 in which
R⁰ is C(=NH)-NH-X, CH₂-NH-X or NH-X¹;
X is hydrogen, (C₁-C₆)-alkyl, (C₁-C₆)-alkylcarbonyl, (C₁-C₆)-alkoxycarbonyl, (C₁-C₁₈)-alkylcarbonyloxy-(C₁-C₆)-alkoxycarbonyl, optionally substituted (C₆-C₁₄)-arylcarbonyl, optionally substituted (C₆-C₁₄)-aryloxycarbonyl, (C₆-C₁₄)-aryl-(C₁-C₆)-alkoxycarbonyl which can also be substituted in the aryl radical, (R⁵O)₂P(O), cyano, hydroxyl, (C₁-C₆)-alkoxy, (C₆-C₁₄)-aryl-(C₁-C₆)-alkoxy which can also be substituted in the aryl radical, or amino;
X¹ has one of the meanings of X or is R'-NH-C(=N-R"), where R' and R" independently of one another have the meanings of X;
R¹ is methyl, ethyl, propyl, butyl, pentyl, hexyl, isopropyl, isobutyl, isopentyl, neopentyl, isohexyl, 3-methylpentyl, sec-butyl, tert-butyl, tent-pentyl, (C₆-C₁₄)-aryl-(C₁-C₈)-alkyl optionally substituted in the aryl radical or (C₃-C₈)-cycloalkyl;
R², R¹⁰ and R¹¹ independently of one another are hydrogen, (C₁-C₈)-alkyl, optionally substituted (C₆-C₁₄)-aryl, (C₆-C₁₄)-aryl-(C₁-C₈)-alkyl optionally substituted in the aryl radical or (C₃-C₈)-cycloalkyl;
R⁵ is hydrogen, (C₁-C₈)-alkyl, optionally substituted (C₆-C₁₄)-aryl or (C₆-C₁₄)-aryl-(C₁-C₈)-alkyl, which can also be substituted in the aryl radical;
R¹² is hydrogen, (C₁-C₈)-alkyl, optionally substituted (C₆-C₁₄)-aryl, (C₆-C₁₄)-aryl-(C₁-C₈)-alkyl optionally substituted in the aryl radical, (C₃-C₈)-cycloalkyl or 2-, 3- or 4-pyridyl;
R¹³ is hydroxyl, (C₁-C₁₈)-alkoxy, (C₆-C₁₄)-aryl-(C₁-C₈)-alkoxy which can also be substituted in the aryl radical, optionally substituted (C₆-C₁₄)-aryloxy, amino or mono- or di((C₁-C₁₈)-alkyl)amino;
n is an integer from 0 to 6;
where an aryl group, which is substituted, carries one, two or three identical or different substituents selected from the group consisting of (C₁-C₈)-alkyl, (C₁-C₈)-alkoxy, halogen, nitro, amino, trifluoromethyl, hydroxyl, methylenedioxy, cyano, hydroxycarbonyl, aminocarbonyl, (C₁-C₄)-alkoxycarbonyl, phenyl, phenoxy, benzyloxy, (R⁵O)₂P(O), (R⁵O)₂P(O)-O- and tetrazolyl.

11. A hydantoin derivative of the formula XVIII 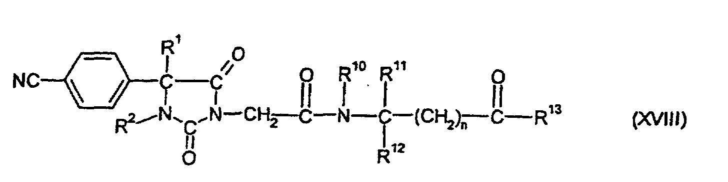 in which
R¹ is methyl, ethyl, propyl, butyl, pentyl, hexyl, isopropyl, isobutyl, isopentyl, neopentyl, isohexyl, 3-methylpentyl, sec-butyl, tert-butyl, tert-pentyl, (C₆-C₁₄)-aryl-(C₁-C₈)-alkyl optionally substituted in the aryl radical or (C₃-C₈)-cycloalkyl;
R², R¹⁰ and R¹¹ independently of one another are hydrogen, (C₁-C₈)-alkyl, optionally substituted (C₆-C₁₄)-aryl, (C₆-C₁₄)-aryl-(C₁-C₈)-alkyl optionally substituted in the aryl radical or (C₃-C₈)-cycloalkyl;
R¹² is hydrogen, (C₁-C₈)-alkyl, optionally substituted (C₆-C₁₄)-aryl, (C₆-C₁₄)-aryl-(C₁-C₈)-alkyl optionally substituted in the aryl radical, (C₃-C₈)-cycloalkyl or 2-, 3- or 4-pyridyl;
R¹³ is hydroxyl, (C₁-C₁₈)-alkoxy, (C₆-C₁₄)-aryl-(C₁-C₈)-alkoxy which can also be substituted in the aryl radical, optionally substituted (C₆-C₁₄)-aryloxy, amino or mono- or di((C₁-C₁₈)-alkyl)amino;
n is an integer from 0 to 6;
where an aryl group, which is substituted, carries one, two or three identical or different substituents selected from the group consisting of (C₁-C₈)-alkyl, (C₁-C₈)-alkoxy, halogen, nitro, amino, trifluoromethyl, hydroxyl, methylenedioxy, cyano, hydroxycarbonyl, aminocarbonyl, (C₁-C₄)-alkoxycarbonyl, phenyl, phenoxy, benzyloxy, (R⁵O)₂P(O), (R⁵O)₂P(O)-O- and tetrazolyl;
or its salts.

12. A hydantoin derivative as claimed in claim 11, in which
R¹ is (C₁-C₄)-alkyl, cyclopropyl or benzyl, preferably methyl;
R² is hydrogen or (C₁-C₄)-alkyl, preferably hydrogen;
R¹⁰ is hydrogen, (C₁-C₆)-alkyl or benzyl, preferably hydrogen;
R¹¹ is hydrogen or (C₁-C₈)-alkyl, preferably hydrogen;
R¹² is optionally substituted (C₆-C₁₄)-aryl or pyridyl, preferably optionally substituted phenyl, particularly preferably unsubstituted phenyl;
R¹³ is hydroxyl or (C₁-C₈)-alkoxy, preferably (C₁-C₄)-alkoxy;
n is an integer from 0 to 3, preferably the numbers 1 or 2, particularly preferably the number 1;
and in which preferably a uniform configuration is present at the asymmetric center in the hydantoin ring and at the carbon atom carrying the radicals R¹¹ and R¹², in particular the S configuration at both,
where an aryl group or phenyl group, which is substituted, carries one, two or three identical or different substituents selected from the group consisting of (C₁-C₈)-alkyl, (C₁-C₈)-alkoxy, halogen, vitro, amino, trifluoromethyl, hydroxyl, methylenedioxy, cyano, hydroxycarbonyl, aminocarbonyl, (C₁-C₄)-alkoxycarbonyl, phenyl, phenoxy, benzyloxy, (R⁵O)₂P(O), (R⁵O)₂P(O)-O- and tetrazolyl.

13. A process for the preparation of hydantoin derivatives as claimed in claim 11 and/or 12, which comprises reacting compounds of the formula Ib 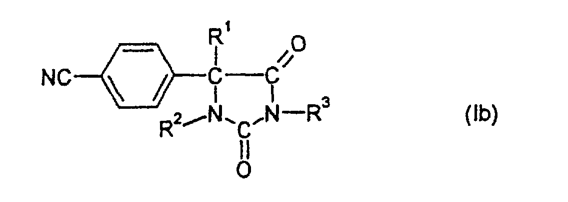 in which R¹ and R² have the meanings given in claim 11 and R³ has the meanings given in claim 1 with the exception of the meaning hydrogen, with compounds of the formula XVII 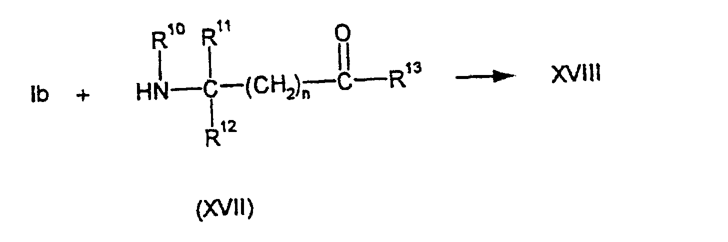 in which R¹⁰ to R¹³ and n have the meanings given in claim 11.

14. The use of hydantoin derivatives as claimed in claim 11 and/or 12 as intermediates for the preparation of pharmaceutical active compounds.

## Revendications

1. Dérivés d'hydantôine de formule générale I 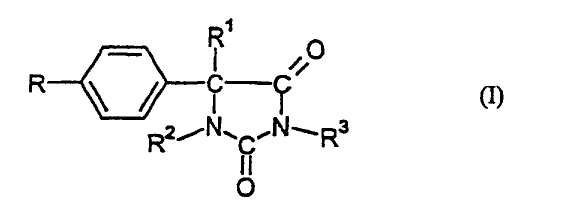 dans laquelle
R représente un groupe cyano, C(=NH)-O-(alkyle en C₁-C₆), C(=NH)-NH-X, CH₂-NH-X ou NH-X¹;
X représente un atome d'hydrogène ou un groupe alkyle en C₁-C₆, (alkyl en C₁-C₆)carbonyle, (alcoxy en C₁-C₆)carbonyle, (alkyl en C₁-C₁₈)carbonyloxy-(alcoxy en C₁-C₆)carbonyle, (aryl en C₆-C₁₄)carbonyle éventuellement substitué, (aryloxy en C₆-C₁₄)carbonyle éventuellement substitué, (aryl en C₆-C₁₄)-(alcoxy en C₁-C₆)carbonyle pouvant aussi être substitué sur le reste aryle, (R⁵O)₂P(O), cyano, hydroxy, alcoxy en C₁-C₆, (aryl en C₆-C₁₄)alcoxy en C₁-C₆ pouvant aussi être substitué sur le reste aryle, ou amino,
X¹ a l'une des significations de X ou représente un groupe R'-NH-C(=N-R") dans lequel R' et R" ont indépendamment l'un de l'autre les significations de X;
R¹ représente un groupe méthyle, éthyle, propyle, butyle, pentyle, hexyle, isopropyle, isobutyle, isopentyle, néopentyle, isohexyle, 3-méthylpentyle, sec-butyle, tert-butyle, tert-pentyle, (aryle en C₆-C₁₄)-alkyle en C₁-C₈ éventuellement substitué sur le reste aryle, ou cycloalkyle en C₃-C₈;
R² représente un atome d'hydrogène ou un groupe alkyle en C₁-C₈, aryle en C₆-C₁₄ éventuellement substitué, (aryl en C₆-C₁₄)-alkyle en C₁-C₈ éventuellement substitué sur le reste aryle ou cycloalkyle en C₃-C₈;
R³ représente un atome d'hydrogène ou un groupe CH₂-CO-OR⁴;
R⁴ représente un atome d'hydrogène ou un groupe alkyle en C₁-C₆, (aryl en C₆-C₁₄)-alkyle en C₁-C₆ éventuellement substitué sur le reste aryle ou aryle en C₆-C₁₄ éventuellement substitué;
R⁵ représente un atome d'hydrogène ou un groupe alkyle en C₁-C₈, aryle en C₆-C₁₄ éventuellement substitué ou (aryl en C₆-C₁₄)-alkyle en C₁-C₈ pouvant aussi être substitué sur le reste aryle;
mais dans laquelle, lorsque R³ représente un atome d'hydrogène, R ne peut représenter un groupe CN, NH₂ ou CH₂-NH₂;
et dans laquelle, lorsque les composés de formule générale I se trouvent sous la forme racémique en ce qui concerne le centre asymétrique du cycle hydantoïne et en méme temps R³ représente un groupe méthoxycarbonylméthyle, R¹ un groupe méthyle et R² un atome d'hydrogène ou un groupe méthyle, R ne peut pas représenter un groupe CN ou C(=NH)-OC₂H₅;
et dans laquelle, lorsque les composés de formule générale I se trouvent sous la forme racémique en ce qui concerne le centre asymétrique du cycle hydantoïne et en même temps R³ représente un groupe méthoxycarbonylméthyle ou hydroxycarbonylméthyle, R¹ un groupe méthyle et R² un atome d'hydrogène ou un groupe méthyle, R ne peut pas représenter un groupe NH₂, CH₂-NH₂, C(=NH)-NH₂, tert-butoxycarbonylaminométhyle ou benzyloxycarbonylguanidino;
et dans laquelle, lorsque les composés de formule générale I se trouvent sous la forme racémique en ce qui concerne le centre asymétrique du cycle hydantoïne et en même temps R¹ représente un groupe méthyle et R² et R³ représentent des atomes d'hydrogène, R ne peut pas représenter le groupe NH CO-CH₃;
et dans laquelle un groupe aryle substitué porte un, deux ou trois substituants identiques ou différents de la série des groupes alkyle en C₁-C₈, alcoxy en C₁-C₈, halogéno, nitro, amine, trifluorométhyle, hydroxy, méthylénedioxy, cyano, hydroxycarbonyle, aminocarbonyle, (alcoxy en C₁-C₄)carbonyle, phényle, phénoxy, benzyloxy, (R⁵O)₂P(O), (R⁵O)₂P(O)-O-, tétrazolyle;
et leurs sels.

2. Dérivés d'hydantoïne selon la revendication 1, dans lesquels
R représente un groupe cyano ou C(=NH)-NH-X;
X représente un atome d'hydrogène ou un groupe (alkyl en C₁-C₆)carbonyle, (alcoxy en C₁-C₆)carbonyle ou (aryl en C₆-C₁₄)-(alcoxy en C₁-C₆)carbonyle éventuellement substitué;
R¹ représente un groupe méthyle, éthyle, propyle, butyle, pentyle, hexyle, isopropyle, isobutyle, isopentyle, néopentyle, isohexyle, 3-méthylpentyle, sec-butyle, tert-butyle, tert-pentyle, (aryle en C₆-C₁₄)-alkyle en C₁-C₈ éventuellement substitué sur le reste aryle, ou cycloalkyle en C₃-C₈;
R² représente un atome d'hydrogène ou un groupe alkyle en C₁-C₈, phényle éventuellement substitué, phényl(alkyle en C₁-C₈) éventuellement substitué sur le reste phényle ou cycloalkyle en C₃-C₈;
R³ représente un groupe CH₂-CO-OR⁴;
R⁴ représente un atome d'hydrogène ou un groupe alkyle en C₁-C₆, (aryl en C₆-C₁₄)-alkyle en C₁-C₆ éventuellement substitué sur le reste aryle ou aryle en C₆-C₁₄ éventuellement substitué;
mais dans lesquels, lorsque les composés de formule générale I se trouvent sous la forme racémique en ce qui concerne le centre asymétrique du cycle hydantoïne et en même temps R³ représente un groupe méthoxycarbonylméthyle, R¹ un groupe méthyle et R² un atome d'hydrogène ou un groupe méthyle, R ne peut pas représenter le groupe CN;
et dans lesquels, lorsque les composés de formule générale I se trouvent sous la forme racémique en ce qui concerne le centre asymétrique du cycle hydantoïne et en même temps R³ représente un groupe méthoxycarbonylméthyle ou hydroxycarbonylméthyle, R¹ un groupe méthyle et R² un atome d'hydrogène ou un groupe méthyle, R ne peut pas représenter le groupe C(=NH)-NH₂;
et dans lesquels un groupe aryle ou phényle substitué est substitué de la manière indiquée dans la revendication 1.

3. Dérivés d'hydantoïne selon la revendication 1 et/ou 2, dans lesquels
R représente un groupe cyano ou C(=NH)-NH₂;
R¹ représente un groupe allyle en C₁-C₄, cyclopropyle ou benzyle, en particulier méthyle;
R² représente un atome d'hydrogène ou un groupe alkyle en C₁-C₄, en particulier un atome d'hydrogène;
R³ représente un groupe CH₂-COOH ou CH₂-COO-(alkyle en C₁-C₄), en particulier CH₂-COOH;
mais dans lesquels, lorsque les composés de formule générale I se trouvent sous la forme racémique en ce qui concerne le centre asymétrique du cycle hydantoïne et en même temps R³ représente un groupe méthoxycarbonylméthyle, R¹ un groupe méthyle et R² un atome d'hydrogène ou un groupe méthyle, R ne peut pas représenter le groupe CN;
et dans lesquels, lorsque les composés de formule générale I se trouvent sous la forme racémique en ce qui concerne le centre asymétrique du cycle hydantoïne et en même temps R³ représente un groupe méthoxycarbonylméthyle ou hydroxycarbonylméthyle, R¹ un groupe méthyle et R² un atome d'hydrogène ou un groupe méthyle, R ne peut pas représenter le groupe C(=NH)-NH₂.

4. Dérivés d'hydantoïne selon l'une ou plusieurs des revendications 1 à 3, **caractérisés en ce qu'**ils se présentent sous une forme énantiomère pure, de préférence sous la configuration S, en ce qui concerne le centre asymétrique du cycle hydantoïne.

5. Procédé de préparation de dérivés d'hydantoïne selon l'une ou plusieurs des revendications 1 à 4, **caractérisé en ce que** l'on fait réagir des esters d'aminoacides de formule générale V 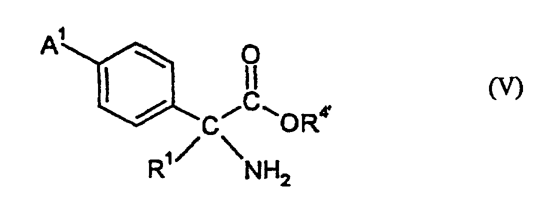 dans laquelle A¹ représente un halogène, de préférence le brome, ou un groupe nitro, R¹ a la signification indiquée dans la revendication 1 et R^{4'} a la signification indiquée dans la revendication 1 pour R⁴ à l'exception de l'hydrogène, avec des esters de l'acide isocyanatoacétique, et on cyclise les produits obtenus pour obtenir les acides hydantoïne-acétiques de formule générale VIII 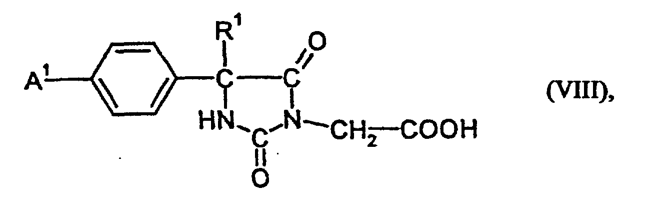 que l'on transforme en les composés de formule générale I en transformant le groupe A¹ en le reste R, le groupe NH en le groupe N-R² et le groupe COOH en le groupe COOR⁴ des composés de formule générale I, ou, lorsque l'on veut préparer des composés de formule générale I dans lesquels R³ représente un atome d'hydrogène, on transforme les composés de formule générale V en les dérivés d'hydantoïne de formule générale III 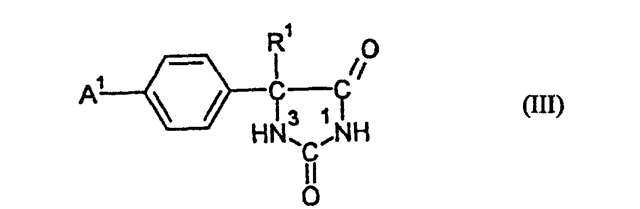 dans lesquels on transforme le groupe A¹ et la fonction N³H.

6. Procédé selon la revendication 5, **caractérisé en ce que** l'on prépare des dérivés d'hydantoïne qui sont des énantiomères purs et ont de préférence la configuration S en ce qui concerne le centre asymétrique du cycle hydantoïne.

7. Procédé de préparation de dérivés d'hydantoine selon l'une ou plusieurs des revendications 1 à 4, **caractérisé en ce que** l'on fait réagir des amides d'aminoacides de formule générale XI 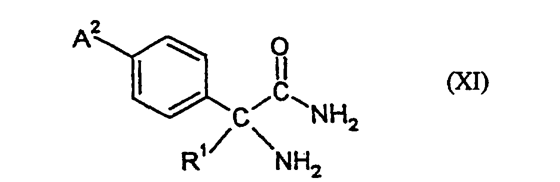 dans laquelle A² représente un halogène, de préférence le brome, ou un groupe nitro ou cyano, et R¹ a la signification donnée dans la revendication 1, avec des esters de l'acide isocyanatoacétique et on cyclise les produits obtenus pour obtenir les acides hydantoïne-acétiques de formule générale XIII 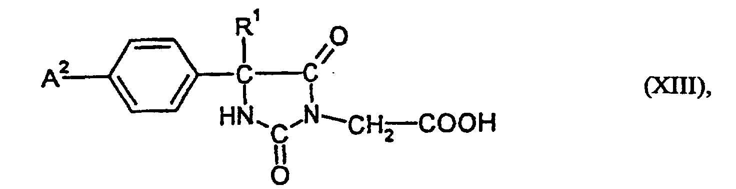 que l'on transforme en les composés de formule générale I en transformant le groupe A² en le reste R, le groupe NH en le groupe N-R² et le groupe COOH en le groupe COOR⁴ des composés de formule générale I, ou, lorsque l'on veut préparer des composés de formulé générale I dans lesquels R³ représente un atome d'hydrogène, on transforme les composés de formule générale XI en les dérivés d'hydantoïne de formule générale XIV 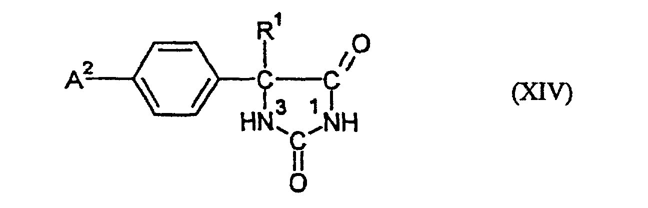 dans lesquels on transforme le groupe A² et la fonction N³H.

8. Procédé selon la revendication 7, **caractérisé en ce que** l'on prépare des dérivés d'hydantoïne qui sont des énantiomères purs et ont de préférence la configuration S en ce qui concerne le centre asymétrique du cycle hydantoïne.

9. Utilisation de dérivés d'hydantoïne selon l'une ou plusieurs des revendications 1 à 4 comme produits intermédiaires pour la préparation de substances actives pharmaceutiques.

10. Utilisation selon la revendication 9, **caractérisée en ce que** l'on prépare des substances actives de formule générale XVI 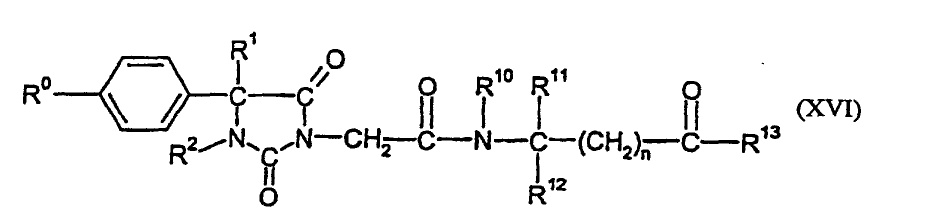 dans laquelle
R⁰ représente un groupe C(=NH)-NH-X, CH₂-NH-X ou NH-X¹;
X représente un atome d'hydrogène ou un groupe allyle en C₁-C₆, (alkyl en C₁-C₆)carbonyle, (alcoxy en C₁-C₆)carbonyle, (alkyl en C₁-C₁₈)carbonyloxy-(alcoxy en C₁-C₆)carbonyle, (aryl en C₆-C₁₄)carbonyle éventuellement substitué, (aryloxy en C₆-C₁₄)carbonyle éventuellement substitué, (aryl en C₆-C₁₄)-(alcoxy en C₁-C₆)carbonyle pouvant aussi être substitué sur le reste aryle, (R⁵O)₂P(O), cyano, hydroxy, alcoxy en C₁-C₆, (aryl en C₆-C₁₄)-alcoxy en C₁-C₆ pouvant aussi être substitué sur le reste aryle, ou amino,
X¹ a l'une des significations de X ou représente un groupe R'-NH-C(=N-R") dans lequel R' et R" ont indépendamment l'un de l'autre les significations de X;
R¹ représente un groupe méthyle, ëthyle, propyle, butyle, pentyle, hexyle, isopropyle, isobutyle, isopentyle, néopentyle, isohexyle, 3-méthylpentyle, sec-butyle, tert-butyle, tert-pentyle, (aryle en C₆-C₁₄)-alkyle en C₁-C₈ éventuellement substitué sur le reste aryle, ou cycloalkyle en C₃-C₈;
R², R¹⁰ et R¹¹ représentent indépendamment les uns des autres un atome d'hydrogène ou un groupe alkyle en C₁-C₈, aryle en C₆-C₁₄ éventuellement substitué, (aryl en C₆-C₁₄)-alkyle en C₁-C₈ éventuellement substitué sur le reste aryle ou cycloalkyle en C₃-C₈;
R⁵ représente un atome d'hydrogène ou un groupe alkyle en C₁-C₈, aryle en C₆-C₁₄ éventuellement substitué ou (aryl en C₆-C₁₄)-alkyle en C₁-C₈ pouvant aussi être substitué sur le reste aryle;
R¹² représente un atome d'hydrogène ou un groupe alkyle en C₁-C₈, aryle en C₆-C₁₄ éventuellement substitué, (aryl en C₆-C₁₄)-alkyle en C₁-C₈ éventuellement substitué sur le reste aryle, cycloalkyle en C₃-C₈ ou 2-, 3- ou 4-pyridyle;
R¹³ représente un groupe hydroxy, alcoxy en C₁-C₁₈, (aryl en C₆-C₁₄)-alcoxy en C₁-C₈ pouvant aussi être substitué sur le reste aryle, aryloxy en C₆-C₁₄ éventuellement substitué, amino ou mono- ou di(alkyl en C₁-C₁₈)amino;
n représente un nombre entier de 0 à 6;
et dans laquelle un groupe aryle substitué porte un, deux ou trois substituants identiques ou différents de la série des groupes alkyle en C₁-C₈, alcoxy en C₁-C₈, halogéno, nitro, amino, trifluorométhyle, hydroxy, méthylènedioxy, cyano, hydroxycarbonyle, aminocarbonyle, (alcoxy en C₁-C₄)carbonyle, phényle, phénoxy, benzyloxy, (R⁵O)₂P(O), (R⁵O)₂P(O)-O-, tétrazolyle.

11. Dérivés d'hydantoïne de formule générale XVIII 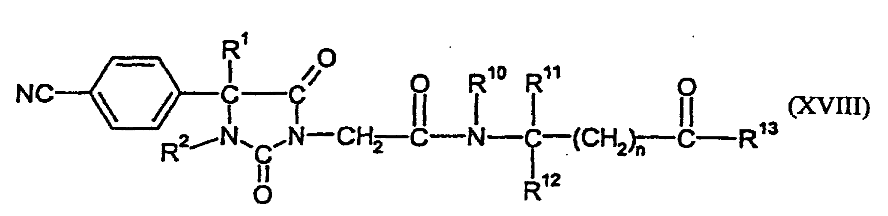 dans laquelle
R¹ représente un groupe méthyle, éthyle, propyle, butyle, pentyle, hexyle, isopropyle, isobutyle, isopentyle, néopentyle, isohexyle, 3-méthylpentyle, sec-butyle, tert-butyle, tert-pentyle, (aryle en C₆-C₁₄)-alkyle en C₁-C₈ éventuellement substitué sur le reste aryle, ou cycloalkyle en C₃-C₈;
R², R¹⁰ et R¹¹ représentent indépendamment les uns des autres un atome d'hydrogène ou un groupe alkyle en C₁-C₈, aryle en C₆-C₁₄ éventuellement substitué, (aryl en C₆-C₁₄)-alkyle en C₁-C₈ éventuellement substitué sur le reste aryle ou cycloalkyle en C₃-C₈;
R¹² représente un atome d'hydrogène ou un groupe alkyle en C₁-C₈, aryle en C₆-C₁₄ éventuellement substitué, (aryl en C₆-C₁₄)-alkyle en C₁-C₈ éventuellement substitué sur le reste aryle, cycloalkyle en C₃-C₈ ou 2-, 3- ou 4-pyridyle;
R¹³ représente un groupe hydroxy, alcoxy en C₁-C₁₈, (aryl en C₆-C₁₄)-alcoxy en C₁-C₈ pouvant aussi être substitué sur le reste aryle, aryloxy en C₆-C₁₄ éventuellement substitué, amino ou mono- ou di(alkyl en C₁-C₁₈)amino;
n représente un nombre entier de 0 à 6;
et dans laquelle un groupe aryle substitué porte un, deux ou trois substituants identiques ou différents de la série des groupes alkyle en C₁-C₈, alcoxy en C₁-C₈, halogéno, nitro, amino, trifluorométhyle, hydroxy, méthylènedioxy, cyano, hydroxycarbonyle, aminocarbonyle, (alcoxy en C₁-C₄)carbonyle, phényle, phénoxy, benzyloxy, (R⁵O)₂P(O), (R⁵O)₂P(O)-O-, tétrazolyle;
et leurs sels.

12. Dérivés d'hydantoïne selon la revendication 11, dans lesquels
R¹ représente un groupe alkyle en C₁-C₄, cyclopropyle ou benzyle, de préférence méthyle;
R² représente un atome d'hydrogène ou un groupe allyle en C₁-C₄, de préférence un atome d'hydrogène;
R¹⁰ représente un atome d'hydrogène ou un groupe alkyle en C₁-C₆ ou benzyle, de préférence un atome d'hydrogène;
R¹¹ représente un atome d'hydrogène ou un groupe alkyle en C₁-C₈, de préférence un atome d'hydrogène;
R¹² représente un groupe aryle en C₆-C₁₄ éventuellement substitué ou pyridyle, de préférence phényle éventuellement substitué, de façon particulièrement préférée phényle non substitué;
R¹³ représente un groupe hydroxy ou alcoxy en C₁-C₈, de préférence alcoxy en C₁-C₄;
n représente un nombre entier de 0 à 3, de préférence le nombre 1 ou 2, de façon particulièrement préférée le nombre 1;
et dans laquelle il y a de préférence une configuration unique au niveau du centre asymétrique du cycle hydantoïne ainsi qu'au niveau de l'atome de carbone portant les restes R¹¹ et R¹², en particulier la configuration S aux deux,
et dans laquelle un groupe aryle ou phényle substitué porte un, deux ou trois substituants identiques ou différents de la série des groupes alkyle en C₁-C₈, alcoxy en C₁-C₈, halogéno, nitro, amino, trifluorométhyle, hydroxy, méthylènedioxy, cyano, hydroxycarbonyle, aminocarbonyle, (alcoxy en C₁-C₄)carbonyle, phényle, phénoxy, benzyloxy, (R⁵O)₂P(O), (R⁵O)₂P(O)-O-, tétrazolyle.

13. Procédé de préparation de dérivés d'hydantoine selon la revendication 11 et/ou 12, **caractérisé en ce que** l'on fait réagir des composés de formule générale Ib 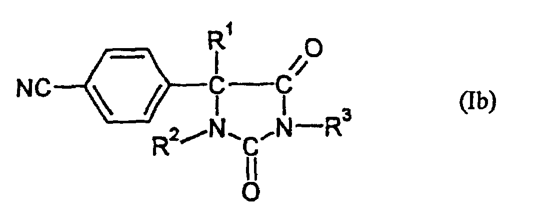 dans laquelle R¹ et R² ont la signification donnée dans la revendication 11 et R³ la signification donnée dans la revendication 1 à l'exception de l'hydrogène, avec des composés de formule générale XVII 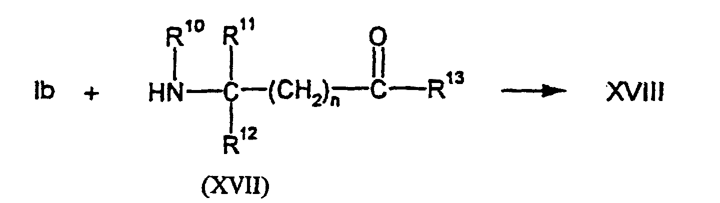 dans laquelle R¹⁰ à R¹³ et n ont la signification donnée dans la revendication 11.

14. Utilisation de dérivés d'hydantoïne selon la revendication 11 et/ou la revendication 12 comme produits intermédiaires pour la préparation de substances actives pharmaceutiques.
